(19) Europäisches Patentamt
European Patent Office
Office européen des brevets

(11) Publication number: **0 298 652 B1**

# (12) EUROPEAN PATENT SPECIFICATION

(45) Date of publication of patent specification: **18.01.95**

(51) Int. Cl.⁶: **C07J 75/00**, C07J 73/00, C07J 51/00

(21) Application number: **88305926.3**

(22) Date of filing: **27.06.88**

## (54) Dehydrogenation process and intermediates.

(30) Priority: **29.06.87 US 67568**
**29.06.87 US 67572**

(43) Date of publication of application:
**11.01.89 Bulletin 89/02**

(45) Publication of the grant of the patent:
**18.01.95 Bulletin 95/03**

(84) Designated Contracting States:
**AT BE CH DE ES FR GB GR IT LI LU NL SE**

(56) References cited:
**EP-A- 0 004 949**
**EP-A- 0 155 096**
**DE-A- 1 805 857**
**GB-A- 2 018 257**

(73) Proprietor: **MERCK & CO. INC.**
**126, East Lincoln Avenue**
**P.O. Box 2000**
**Rahway**
**New Jersey 07065-0900 (US)**

(72) Inventor: **Bhattacharya, Apurba**
**276 Waite Avenue-4**
**Rahway**
**New Jersey 07065 (US)**

Inventor: **Douglas, Alan W.**
**1760 Sand Hill Road**
**Monmouth Junction**
**New Jersey 08852 (US)**
Inventor: **Grabowski, Edward J.**
**741 Marcellus Drive**
**Westfield**
**New Jersey 07090 (US)**
Inventor: **Dolling, Ulf H.**
**641 Fourth Avenue**
**Westfield**
**New Jersey 07090 (US)**

(74) Representative: **Cole, William Gwyn et al**
**European Patent Department**
**Merck & Co., Inc.**
**Terlings Park**
**Eastwick Road**
**Harlow**
**Essex CM20 2OR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention is concerned with a process for dehydrogenating steroids, particularly 3-oxo-4-azasteroids, to prepare corresponding $\Delta^1$ compounds and novel intermediates utilized in the process.

Heretofore, azasteroids have been dehydrogenated to introduce a $\Delta^1$ double bond by means of benzene seleninic anhydride oxidation in which the saturated compound was heated with the benzene seleninic anhydride in refluxing chlorobenzene. Back, T. G., J. Org. Chem. 46: 1442 (1981); Rasmussen et al., J. Med. Chem. 29: 2298 (1986). Additionally, sulfoxide elimination has been a process used to accomplish the dehydrogenation. See U.S. Patent 4,377,584, 4,220,775 and EP application 85301122.9 (published September 18, 1985). However, these reactions have been found to give poor yields, with a high degree of impurities and one requires the use of a selenium catalyst which is very expensive and is quite toxic.

It has also been known to dehydrogenate a 3-oxo-4-azalactam by a complicated 5-step process which involves a sulfenate intermediate. See Magnus et al., J. Am. Chem. Soc. 1986: 108 212-217. However, the process of the present invention provides a versatile single-pot process for the direct introduction of a $\Delta^1$ double bond into 3-oxo-4-azasteroids in high yields and without the attendant toxicity and impurity problems associated with the prior art.

DE-A-1805857 describes a process for introducing a 1,2 double bond into a steroid nucleus utilising a quinone, for example 2,3-dichloro-5,6-dicyanobenzoquinone (DDQ), as an oxidising agent.

GB-A-2018257 describes a process whereby two double bonds are introduced into a steroid nucleus at the 1,2 and 4,5 positions, either side of a 3-oxo functionality, with DDQ being specified as the oxidising agent in this transformation.

The process of the present invention is a single-pot silylation mediated quinone oxidation of lactones and lactams, in particular 3-keto-4-azasteroids to the corresponding $\Delta^1$ double bond compound. The present invention provides a unique way to dehydrogenate a wide variety of compounds while avoiding the disadvantages of the prior art methods of effecting such transformations. The disadvantages overcome by the present invention include complicated multi-step processes, poor yields, unwanted by-products and the use of toxic selenium catalysts.

The present invention provides a process for dehydrogenating a compound of the formula

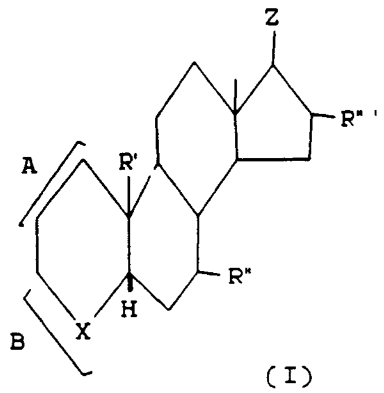

(I)

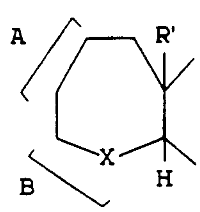

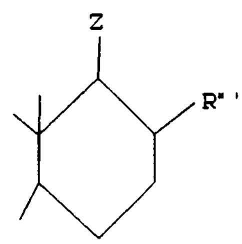

(II) (III)

where Formula (I) may alternatively have the structure of partial Formulas (II) and/or (III); wherein, A is

(1)-$CH_2$-$CH_2$-;

(2)

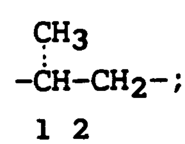

B is

(1)

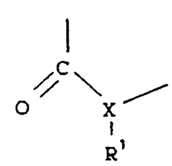

where X is N or O; and

$R^1$ is absent or is

(a) hydrogen;

(b) methyl or ethyl;

(c) $NR^2R^3$ where $R^2$ and $R^3$ are hydrogen or methyl; or

(d) cyano; or

(2)

(a)

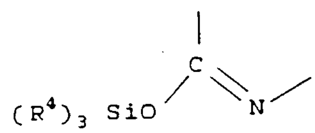

(b)

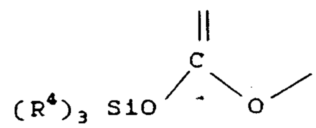

(c)

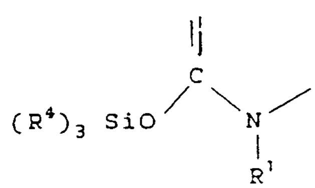

where $R^4$ is any of methyl, $C_{1-8}$ straight or branched chain alkyl, $C_{3-6}$ cycloalkyl, phenyl or combinations thereof;

R' is hydrogen or methyl;

R'' is hydrogen or $\beta$-methyl;

R''' is hydrogen, $\beta$-methyl or hydroxyl;

Z is

(1) $\beta$-hydrogen and $\alpha$-hydroxyl;

(2) $\alpha$-hydrogen or $\alpha$-hydroxyl and

(a)

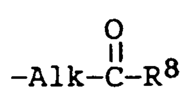,

where Alk is present or absent and is a straight or branched hydrocarbon chain of 1 to 12 carbon atoms; and $R^8$ is,

(i) hydrogen,

(ii) hydroxyl,

(iii) $C_{1-12}$ alkyl,

(iv) $NR^9R^{10}$, where $R^9$ and $R^{10}$ are each independently selected from hydrogen; $C_{1-12}$ straight or branched chain alkyl; $C_{1-12}$ straight or branched chain alkyl having a hydrogen substituted with a hydroxy, carboxylic acid or $C_{1-4}$ alkyl ester; $C_{3-6}$ cycloalkyl; phenyl; or $R^9$ and $R^{10}$ taken together with the nitrogen to which they are attached represent a 5-6 membered saturated ring comprising up to one other heteroatom selected from oxygen and nitrogen; or

(v) $OR^{11}$, where $R^{11}$ is M, where M is hydrogen or alkali metal, or $C_{1-18}$ straight or branched chain alkyl; benzyl; or

(b) -(Alk)-$OR^{12}$, where Alk is present or absent and has the same meaning as above; and $R^{12}$ is

(i) phenyl $C_{1-6}$ alkylcarbonyl,

(ii) $C_{5-10}$ cycloalkylcarbonyl,

(iii) benzoyl, or

(iv) $C_{1-18}$ alkoxycarbonyl;

(v) hydrogen;

(3)

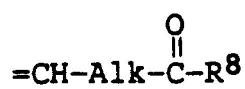

or =CH-Alk-$OR^{12}$, where Alk is present or absent and has the same meaning as above, and $R^8$ and $R^{12}$ have the same meaning as above, and $R^{12}$ is also hydrogen or $C_{1-20}$ alkylcarbonyl;

(4)

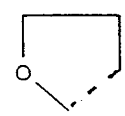

where the dashed bond replaces the 17$\alpha$ hydrogen;

(5) $\alpha$-hydrogen and

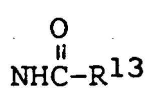,

where $R^{13}$ is,

(a) $C_{1-12}$ alkyl; or

(b) $NR^9R^{10}$;

(6) $\alpha$-hydrogen and cyano; or

(7) $\alpha$-hydrogen and tetrazolyl;

which comprises reacting the compound with a silylating agent in the presence of a quinone to introduce a $\Delta^1$ double bond.

The azasteroid compounds prepared by the processes of the present invention are testosterone-5$\alpha$-reductase inhibitors useful for treating the hyperandrogenic conditions of acne vulgaris, seborrhea, female hirsutism, androgenic alopecia including male pattern alopecia, prostatic carcinoma and benign prostatic

hypertrophy by topical or systemic administration.

Novel silylated intermediate compounds useful in preparing the corresponding $\Delta^1$ compound are also an important part of the present invention.

Generally, the process of the invention involves treatment of the saturated starting lactam, lactone or azasteroid with a silyating agent in the presence of a quinone. A number of silylating agents capable of silylating lactams, lactones and azasteroids can be used. For example, bistrimethylsilylacetamide, bistrimethylsilyltrihaloacetamide, hexamethyldisilazane or bistrimethylsilylurea are silyating agents that can be used in the processes of the present invention. The bistrimethylsilyltrihaloacetamide silyating agent can have any halo group as a moiety thereof, such as chloro, fluoro, bromo or iodo. The preferred silyating agent is bistrimethylsilyltrifluoroacetamide (BSTFA).

Primarily, the readily available 2,3-dichloro-5,6-dicyano-1,4-benzoquinone (DDQ) and 3,4,5,6-tetrachloro-1,2-benzoquinone are preferred for use in the process of the present invention, but any other quinone of sufficient reaction potential can also be used. For example, ortho- or para-benzoquinones of the following formula:

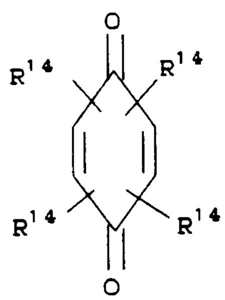

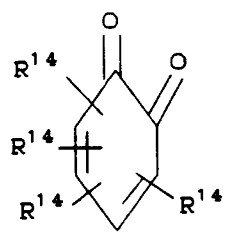

where $R^{14}$ is hydrogen, $C_{1-6}$ alkyl, $C_{1-6}$ alkoxy, halo such as Cl, F, I, Br, nitro or cyano, can be used in the present invention. This would include quinones such as 2,3,5-trimethyl-1,4-benzoquinone, 2,6-dimethyl-1,4-benzoquinone, 2,6-di-t-butyl-1, 4-benzoquinone, 3,5-di-t-butyl-1,2-benzoquinone and 2,3-di-alkoxy-1,4-benzo-quinone. Other quinones of sufficient reaction potential can be substituted for DDQ and still be within the scope of the present invention.

As a general procedure, a lactam, lactone or 3-keto-4-azasteroid is treated with a quinone and a silyating agent in aliphatic or cyclic ethers, or chlorinated or aromatic hydrocarbon solvents with or without strong acid catalysis and with or without preliminary low temperature aging and then, first at a lower temperature, and then at elevated temperatures under nitrogen for 5 to 25 hours to cleanly produce the corresponding dehydrogenated lactam, or lactone in high yield. These conditions were also used to convert a 3-keto-4-azasteroid to the corresponding $\Delta^1$-azasteroid with consistently high yields.

The reaction scheme can be represented as follows:

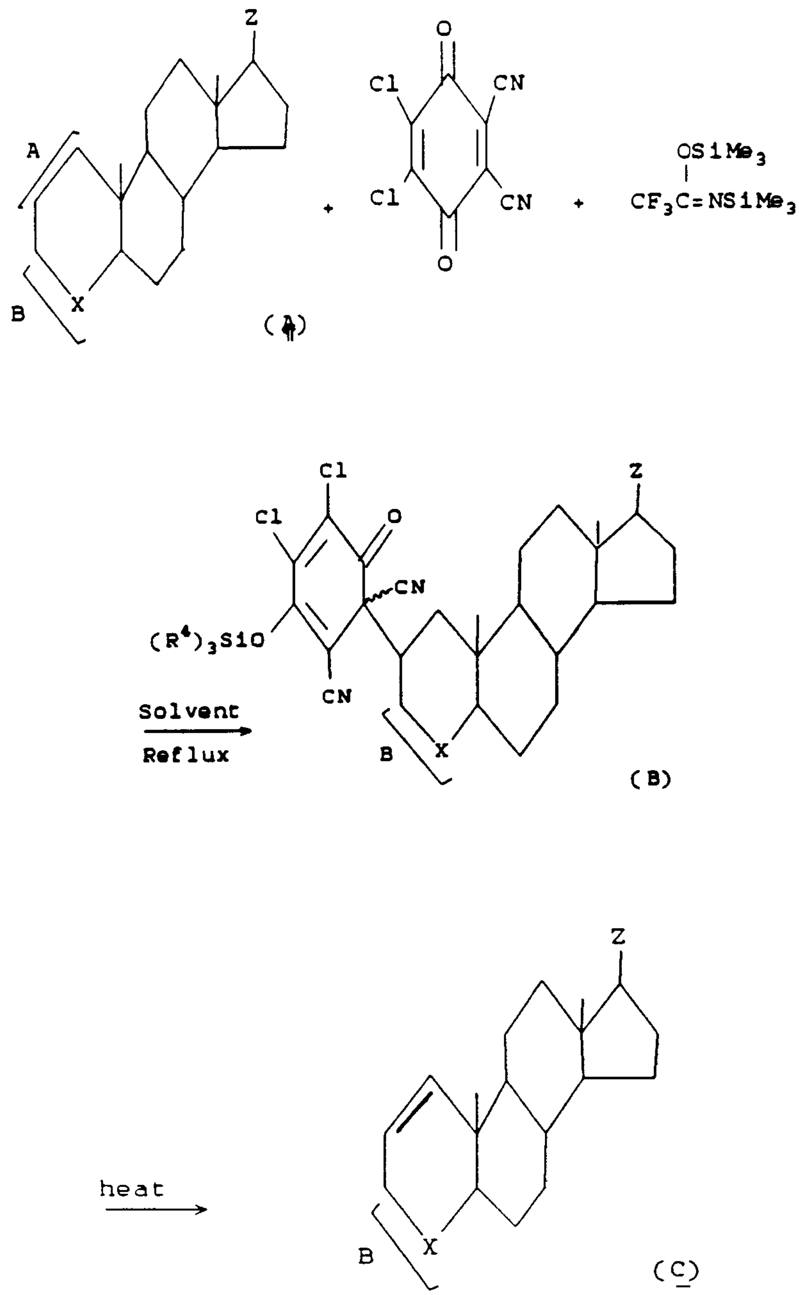

(A) (B) (C)

When X is $NR^1$ or O, the compound of A is reacted with DDQ and BSTFA to form a diastereomeric intermediate B of the following partial structure:

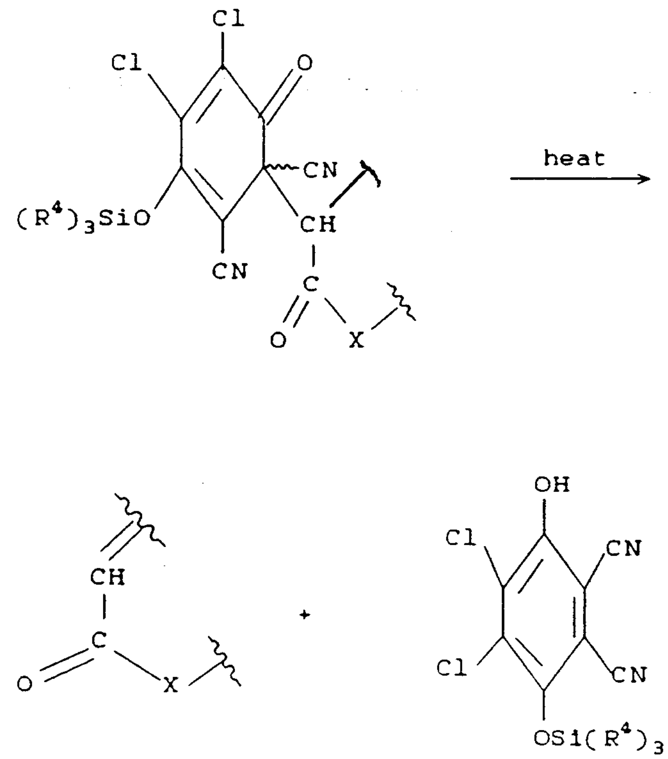


Heating the diastereomeric mixture B results in formation of the corresponding $\Delta^1$ compound. Cyclohexane-1,3-dione is used to decompose the residual DDQ prior to thermolysis.

When X is N, the compound A is reacted with DDQ and BSTFA to form an intermediate according to the following partial structure:

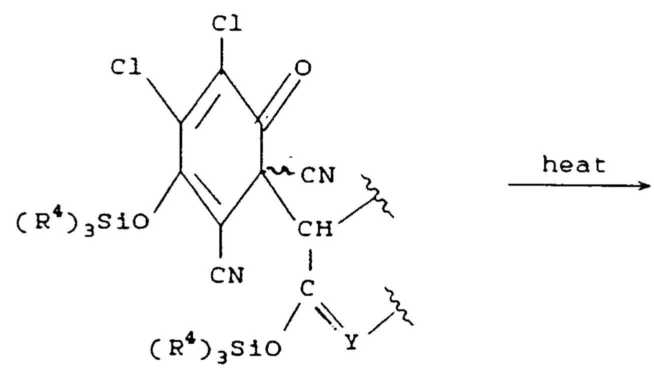

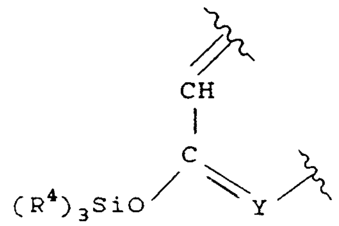

+

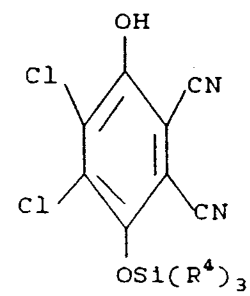

Y = N

For example, the 17$\beta$-carboxy-4-aza-androstan-3-one (A) is reacted with dichlorodicyano-p-benzoquinone (DDQ) and bistrimethyl-silyltrifluoro-acetamide (BSTFA) to form a diastereomeric intermediate (B). The intermediate (B) is subjected to dioxane reflux under heat to form the 17$\beta$-carboxy-3-oxo-4-substituted-4-aza-androst-1-ene-3-one (C) corresponding to formula I.

The novel chemical intermediates formed in the process disclosed herein is also a significant part of the present invention. These novel chemical intermediates (B) are formed as diastereomeric mixtures, however, each diastereomer is included within the scope of the invention. The intermediates are compounds of the formula:

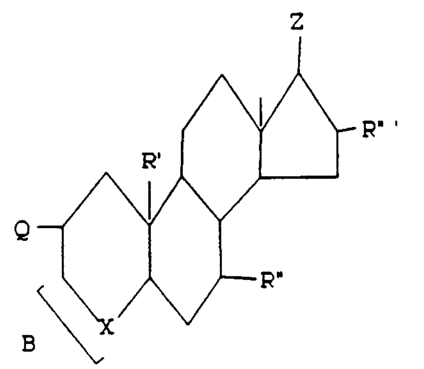

(I)

wherein:
Q is absent or is

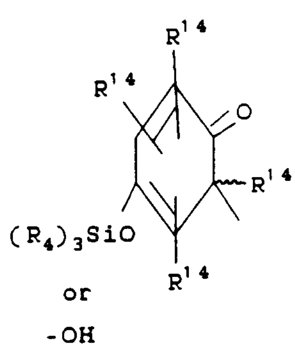

or

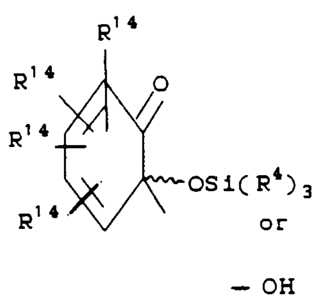

where $R^4$ is methyl, $C_{1-8}$ straight or branched chain alkyl, $C_{3-6}$ cycloalkyl, phenyl, or combinations thereof; and $R^{14}$ is hydrogen, $C_{1-6}$ straight or branched alkyl, $C_{1-6}$ alkoxy, halo, nitro or cyano;

B is

(a) when Q is present,

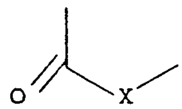

where X is $NR^1$ or O; and $R^1$ is absent or is hydrogen, methyl or ethyl;

(b)

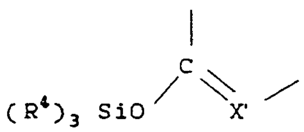

where X' is N;

(c)

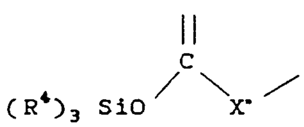

where X" is O;

Z is

(1) $\beta$-hydrogen and $\alpha$-hydroxyl;

(2) $\alpha$-hydrogen or $\alpha$-hydroxyl and

(a)

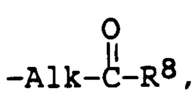

where Alk is present or absent and is a straight or branched hydrocarbon chain of 1 to 12 carbon atoms; and $R^8$ is,

(i) hydrogen,

(ii) hydroxyl,

(iii) $C_{1-12}$ alkyl

(iv) $NR^9R^{10}$, where $R^9$ and $R^{10}$ are each independently selected from hydrogen; $C_{1-12}$ straight or branched chain alkyl; $C_{1-12}$ straight or branched chain alkyl having a hydrogen substituted with a hydroxy, carboxylic acid or $C_{1-4}$ alkyl ester; $C_{3-6}$ cycloalkyl; phenyl; or $R^9$ and $R^{10}$ taken together

with the nitrogen to which they are attached represent a 5-6 membered saturated ring comprising up to one other heteroatom selected from oxygen and nitrogen; or

(v) $OR^{11}$, where $R^{11}$ is M, where M is hydrogen or alkali metal, or $C_{1-18}$ straight or branched chain alkyl; benzyl; or

(b) $-(Alk)-OR^{12}$, where Alk is always present and has the same meaning as above; and $R^{12}$ is

(i) phenyl $C_{1-6}$ alkylcarbonyl.

(ii) $C_{5-10}$ cycloalkylcarbonyl,

(iii) benzoyl, or

(iv) $C_{1-18}$ alkoxycarbonyl;

(v) amino, or $C_{1-8}$ alkyl substituted amino, carbonyl; or

(vi) hydrogen, provided that Alk is a branched $C_3$-$C_8$ chain;

(3)

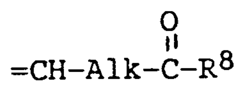

or $=CH-Alk-OR^{12}$, where Alk is present or absent and has the same meaning as above, and $R^8$ and $R^{12}$ have the same meaning as above, and $R^{12}$ is also hydrogen or $C_{1-20}$ alkylcarbonyl;

(4)

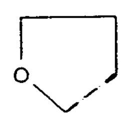

where the dashed bond replaces the 17α hydrogen;

(5) a-hydrogen and

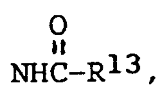

where $R^{13}$ is,

(a) $C_{1-12}$ alkyl; or

(b) $NR^9R^{10}$;

(6) α-hydrogen and cyano; or

(7) α-hydrogen and tetrazolyl;

R' is hydrogen or methyl;

R'' is hydrogen or β-methyl;

R''' is hydrogen, β-methyl or hydroxyl.

The following examples should be considered as not limiting the invention and will serve to illustrate the manner in which the present invention is accomplished.

EXAMPLE 1

3-Oxo-4-aza-5α-androst-1-ene-17β-carboxylic acid

A 1 l three neck round bottom flask equipped with a nitrogen inlet, reflux condenser, addition funnel, mechanical stirrer and an immersion thermometer was charged with 180 mL dioxane followed by 18 g of 3-oxo-4-aza-5α-androstan-17β-carboxylic acid portionwise with stirring. To the stirred suspension was added portionwise 13.86 g of DDQ. The flask was evacuated (22" Hg) and flushed with nitrogen three times. To this stirred suspension was added BSTFA via the addition funnel at the rate of 50 mL/minute. The temperature slowly went up form 22° to 25° in a period of thirty minutes as most of the solids dissolved within this period to afford a clear solution.

The solution was stirred for 18 hours at 22° after which time formation of the two diastereomeric adducts were observed.

To the solution was added 0.54 g cyclohexane-1,3-dione and the reaction mixture was stirred at 22° for an additional three hours to decompose any residual DDQ. The solution was then heated in an oil bath so that a very gentle reflux was maintained. (Bath temperature 120°, Internal Temperature 108°) After refluxing for 20 hours complete disappearance of the adducts and formation of $W^1$ acid were observed.

The reaction mixture was cooled to 22° and poured slowly over a period of 2 minutes into a stirred mixture of 300 mL $CH_2Cl_2$ and 60 mL 1% aqueous sodium bisulfite solution.

The flask was rinsed with 50 mL $CH_2Cl_2$. After the mixture was stirred for thirty minutes, 18 mL 6N HCl was added to this mixture and stirred for an additional thirty minutes. The heterogeneous mixture was filtered and the residue was washed with 60 mL 1N HCl followed by 250 mL $CH_2Cl_2$. The filtrated was transferred into a seperatory funnel and allowed to settle. The bottom $CH_2Cl_2$ layer containing the product was separated from the top aqueous layer and the top layer was washed with 60 mL $CH_2Cl_2$. The combined $CH_2Cl_2$ layer was washed with 200 mL 2N HCl. The aqueous layer was washed with 60 mL $CH_2Cl_2$. The two $CH_2Cl_2$ solutions were combined. The liquid chromatography yield at this stage was 16.0 g (88%). The combined $CH_2Cl_2$ solution was then distilled and concentrated to about 120 mL volume. The distillation was continued and acetonitrile was added to the flask at such a rate so that the total volume was maintained at approximately 120 mL. After addition of about 400 mL acetonitrile the distillation head showed a temperature of about 82°. Gas chromatography analysis performed on the mother liquor showed the presence of about 2% dioxane. The distillation was discontinued at this point. The suspension was cooled to 20° and aged at 20° for 20 hours with stirring. The crystals were filtered and washed with acetonitrile until the filtrate was colorless. Approximately 100 mL acetonitrile was used for the wash. The wet cake was dried at 60°C under vacuum (about 1mm mg) overnight to produce 15.3 g of the $W^1$ acid.

EXAMPLE 2

17β-(t-butylcarbamoyl)-4-aza-5α-androst-1-ene-3-one

A 50 ml three neck round bottom flask equipped with a nitrogen inlet, reflux condenser, addition funnel, magnetic stirrer and an immersion thermometer was charged with 10 ml dioxane followed by 1 g of 17β-N-(t-butylcarbamoyl)-4-aza-5α-androstan-3-one portionwise with stirring. To the stirred suspension was added portionwise 0.656g DDQ. The flask was evacuated (20" Hg) and flushed with nitrogen three times. To this stirred suspension was added BSTFA via a syringe at the rate of about 2 mL/min. Most of the solids dissolved during a period of half of an hour to afford a clear red solution. The solution was stirred for 18 hours at 22° after which time complete disappearance of standing material and formation of the two diastereometric adducts were observed by liquid chromatography. To this solution was added cyclohexane-1,3-dione and the reaction mixture was stirred at 22° for an additional three hours to decompose any residual DDQ. The solution was then heated in an oil bath so that a very gentle reflux was maintained. After refluxing for 20 hours complete disappearance of the adducts and formation of the title compound were observed by liquid chromatography. The reaction mixture was cooled to 22° and poured slowly into a stirred mixture of 30 ml $CH_2Cl_2$ and 6 mL 1% aqueous sodium bisulfite solution. The flask was rinsed with 10 mL $CH_2Cl_2$. After the mixture was stirred for 15 minutes 4 mL 6N HCl was added to this mixture and stirred for an additional 15 minutes. The heterogeneous mixture was filtered and the residue was washed with 20 mL $CH_2Cl_2$. The filtrate was transferred to a seperatory funnel and allowed to settle. The bottom $CH_2Cl_2$ layer containing the product was separated from the top aqueous layer and the top layer was washed with 20 mL $CH_2Cl_2$. The combined $CH_2Cl_2$ layer was washed with 20 mL 2% sodium hydroxide solution. The $CH_2Cl_2$ solution was then distilled and concentrated to about 6 mL volume. The distillation

was continued and isopropyl acetate was added to the flask at such a rate so that the total volume was maintained at approximately 6 mL. The distillation was discontinued when the distillation head showed a temperature of about 85°. The suspension was cooled at 0° for 6 hours. The crystals were filtered and washed with 2 mL isopropyl acetate. The crystals were dried at 60° under vacuum (1 mm Hg) overnight to produce 0.8 g of the title compound (80%).

EXAMPLE 3

To 250 mg of 17-β-(t-butylcarbamoyl)-4-aza-5α-androstan-3-one in a glass NMR tube were added approximately 2 mL of methylene chloride (as a $CH_2Cl_2CD_2Cl_2$ mixture suitable for NMR studies), 0.7 mL BSTFA and 60 mcL $CF_3SO_3H$. The tube was cooled in methanol-ice and 168 mg tetrachloro-1,2-benzoquinone added accompanied by injection of a stream of dry 2 $N_2$. The tube was then closed with a snugly fitting polyethylene stopper, sealed with stretched Parafilm$^R$; agitated briefly with only momentary warming, and placed in a cooled NMR probe which had been previously adjusted for $^{13}C$ observations of reaction intermediates at approximately-5°C. Spectra were obtained from time to time over a period of two days during which the sample temperature was maintained between zero and -10°C. The solution was then allowed to age five days at ambient temperature while additional sepctra were recorded from time to time. At the end of this aging the entire solution was quantitatively transferred, quenched with 1 mL acetic acid and volumetrically diluted for replicate HPLC analyses which gave 80-84% yield, based on assay vs pure 17-β-(t-butylcarbamoyl)-4-aza-5α-androst-1-ene-3-one, from the saturated lactam precursor.

EXAMPLE 4

A 100 mL three-neck round bottom flask equipped with a nitrogen inlet, reflux condenser, magnetic stirrer and a septum inlet was charged with 17-β-(t-butylcarbamoyl)-4-aza-5α-androstan-3-one (4.0 g), DDQ (4.0 g) and dioxane (26 ml). To this suspension was added BSTFA (10.5 g) with stirring via a syringe over a period of 1 min. Heating the mixture at reflux for 10 minutes afforded a clear solution. The solution was refluxed for 18 hours at the end of which complete disappearance of starting material was observed by LC. The solution was cooled to room temperature and added to a mixture of 100 mL each 5% $NaHSO_3$ and $CH_2Cl_2$, precipitating the hydroquinone which was separated by filtration. The $CH_2Cl_2$ layer of the filtrate was separated, extracted twice with 100 mL of 5% $NaHSO_3$, concentrated to a thick oil and triturated with 200 mL diethyl ether. First and second crop solids were isolated and dried 4 hours at 50° under vacuum yielding a total of 3.28 g of 88% pure 17-β-(t-butylcarbamoyl)-4-aza-5α-androst-1-ene-3-one.

EXAMPLE 5

3-Oxo-4-aza-5α-androst-1-ene-17β-carboxylic acid ($\Delta^1$-Aza acid)

A 1 L 3-neck round bottom flask equipped with a nitrogen inlet, reflux condenser, additional funnel, mechanical stirrer and an immersion thermometer was charged with 320 mL of toluene, 20 g of 3-oxo-4-aza-5α-androstan-17β-carboxylic acid, 15.7 g of DDQ, 70 mL of BSTFA and 0.32 mL of triflic acid. The flask was evacuated and flushed with $N_2$ three times. The reaction mixture was stirred for 18 h at 38-40° after which time complete disappearance of starting material and formation of two diastereomeric adducts was observed by LC. Cyclohexane-1,3-dione (0.8 g) was added and the reaction mixture was stirred at 38-40° for 3 hours to quench any residual DDQ. The solution was then heated to gentle reflux for 20 h after which time complete disappearance of the adducts and formation of the $W^1$-acid (95% assay yield) was observed by LC. The reaction was cooled to 20°C. Aqueous NaOH (430 mL, 1% by wt) and 120 mL of isopropanol were added. The mixture was stirred for 30 min, allowed to settle and the bottom aqueous layer was reextracted with a mixture of 430 mL toluene and 120 mL i-propanol. The combined toluene layers were extracted with 320 mL of 1% NaOH. The bottom aqueous layer containing the product was extracted and the organic layer was washed with another 160 mL of 1% NaOH. The aqueous layers were combined and isopropanol removed by vacuum distillation and replaced with 100 mL of acetonitrile. The mixture was warmed to 60-65°C and acidified with 12 mL of 6N HCl to pH 1, aged at 60-65°C for 2 hours, cooled to 20°C and aged for 18 hours and filtered. The filter cake was washed with 100 mL of $H_20$/ $CH_3CN$ (4/1). The product was dried at 50-60°C under vacuum to yield 17.6 g (88%) of the desired $W^1$-aza acid.

EXAMPLE 6

Methyl-3-oxo-4-aza-5α-androst-1-ene-17β-carboxylate

A 1 L three-neck round bottom flask equipped with a nitrogen inlet, reflux condenser, additional funnel, mechanical stirrer and an immersion thermometer was charged with 320 mL of toluene, 20 g of methyl-3-oxo-4-aza-5α-androstan-17β-carboxylate, 14.73 g of DDQ, 67 mL of BSTFA and 0.32 mL triflic acid. The flask was evacuated and flushed with $N_2$ three times. The reaction mixture was stirred for 18 h at 22° after which time complete disappearance of starting material and formation of the two diastereomeric adducts was observed by LC. Cyclohexane-1,3-dione was added and the reaction mixture was stirred at 22° for 3 hours to quench any residual DDQ. The solution was then heated to gentle reflux for 20 h after which time complete disappearance of the adducts and formation of the methyl-3-oxo-4-aza-5-androst-1-ene-17-carboxylate was observed by LC. The toluene solution was cooled to 22°C and 42 mL $CH_2Cl_2$ and 390 mL saturated sodium bicarbonate solution added with stirring. The bottom layer was separated from the top organic layer containing the product. The top layer was washed once more with 390 mL saturated sodium bicarbonate solution. The toluene solution was distilled to 100 mL volume. Toluene was displaced by n-Butylacetate via distillation maintaining 100 mL volume. The slurry was aged at 22° for 18 hours and filtered to produce the product steroid in 91% yield.

EXAMPLE 7

By following the procedure of the preceding example a 90% yield of 17β-(t-butylcarbamoyl)-4-aza-5α-androst-1-ene-3-one was obtained from the corresponding 17β-(t-butylcarbamayl)-4-aza-5α-androstan-3-one.

EXAMPLE 8

By following the procedure of the preceeding example a 90% yield of 22(R,S)-methyl-4-aza-21-nor-5α-cholen-3,20-dione was obtained from the corresponding 22(R,S)-methyl-4-aza-21-nor-5α-cholan-3,20-dione.

EXAMPLE 9

A 100 mL three neck round bottom flask equipped for magnetic stirring and a nitrogen inlet was charged with 2 g of 17β-(t-butylcarbamoyl)-4-aza-5α-androstan-3-one, 20 mL $CH_2Cl_2$, and 1.344 g of ortho-chloranil. The solution was cooled to -8° by means of methanol/ice cooling and 6 mL BSTFA was added via syringe (ca. 1 min) followed by 0.48 mL triflic acid. The solution was stirred at -10° for 2 days followed by 22° for 6 days, after which time complete disappearance of starting material was observed by LC. At the end of this age HPLC analyses showed 1.6 g of 17β-(t-butylcarbamoyl)-4-aza-5α-androst-1-ene-3-one (based on assay vs pure 17β-(t-butylcarbamoyl)-4-aza-5α-androst-1-ene-3-one; yield 80%.

EXAMPLE 10

Isolation of the diastereomeric adduct formed between 3-oxo-4-aza-5α-androstan-17β-carboxylic acid and DDQ

A 100 mL 3 neck round bottom flask equipped with a nitrogen inlet and magnetic stirring was charged with 20 mL tetrahydropyran, 2g of 3-oxo-4-aza-5α-androstan-17β-carboxylic acid, 6.7 mL of BSTFA, 1.47 g of DDQ and 2 mcL of triflic acid. The solution was stirrd under $N_2$ for 20 hours at 22° after which time complete disappearance of starting material and formation of the two diastereomeric adducts was observed by LC. The solution was cooled to 4° (ice cooling) and 0.2 mL water was added to it. The mixture was stirred at 4° for 3 hours. The solvent was removed at 20° in the rotovapor to afford a yellow oil which was tritiated with 60 mL $CH_2Cl_2$. The two diastereomeric adduct crystallized. The crystals were aged for 1 hour at 22° filtered, washed with 20 mL $CH_2Cl_2$ and dried in vacuum at 22° to afford 90% yield of the two diastereomeric adducts.

EXAMPLE 11

Preparation of 3-oxo-4-aza-5α-androst-1-ene-17β-carboxylic acid via thermolysis of the isolated diasteremeric adducts

100 mg of the two diasteromeric adducts (obtained in Example 10) were heated in 2 mL dioxane under $N_2$ atmosphere for 18 hours to produce 36 mg (by LC analysis, 60%) of the desired 3-oxo-4-aza-5α-androst-1-ene-17β-carboxylic acid.

EXAMPLES 12-37

By following the procedures of Example 1 or 2 but substituting the corresponding 1,2-saturated steroid as a starting material, the following compounds could be obtained.

| Example | Compound Name |
|---|---|
| 12 | 17ß-hydroxy-4-aza-5α-androst-1-en-3-one |
| 13 | 3-oxo-4-aza-5α-pregn-1-ene-20a-carboxylic acid |
| 14 | methyl-3-oxo-4-aza-5α-pregn-1-ene-20a-carboxylate |
| 15 | 2',3'a-tetrahydrofuran-2'-spiro-17-(4-aza-5α-androst-1-en-3-one) |
| 16 | 23-methyl-4-aza-21-nor-5α-chol-1-ene-3,20-dione |
| 17 | 17ß-(2-pyrrylcarbonyl)-4-aza-5α-androst-1-en-3-one |

| Example | Compound Name |
|---|---|
| 18 | 17ß-(t-butyldimethyl-silyloxy)-4-aza-5α-androst-1-en-3-one |
| 19 | 3-oxo-4-aza-5α-androst-1-ene-17ß-carboxamide |
| 20 | N-ethyl-3-oxo-4-aza-5α-androst-1-ene-17ß-carboxamide |
| 21 | N-(2,4,4,-trimethyl-2-pentyl) 3-oxo-4-aza-5α-androst-1-ene-17ß-carboxamide |
| 22 | N,N-bis(2-propyl) 3-oxo-4-aza-5α-androst-1-ene-17ß-carboxamide |
| 23 | 17ß-hydroxy-4-methyl-4-aza-5α-androst-1-en-3-one |
| 24 | 4-methyl-3-oxo-4-aza-5α-pregn-1-ene-20a-carboxylic acid |

| Example | Compound Name |
| --- | --- |
| 25 | methyl 4-methyl-3-oxo-4-aza-5α-pregn-1-20a-carboxylate |
| 26 | 2',3'a-tetrahydrofuran-2'-spiro-17-(4-methyl-4-aza-5α-androst-1-en-3-one) |
| 27 | 4,23-dimethyl-4-aza-21-nor-5α-chol-1-ene-3,20-dione |
| 28 | 4,22-dimethyl-4-aza-21-nor-5α-chol-1-ene-3,20-dione |
| 29 | 4-methyl-17ß-(2-pyrrylcarbonyl)-4-aza-5α-androst-1-en-3-one |
| 30 | 17ß-(t-butyldimethylsilyloxy)-4-methyl-4-aza-5α-androst-1-en-3-one |
| 31 | methyl 4-methyl-3-oxo-4-aza-5α-androst-1-ene-17ß-carboxylate |

| Example | Compound Name |
|---|---|
| 32 | 4-methyl-3-oxo-4-aza-5α-androst-1-ene-17ß-carboxamide |
| 33 | N-ethyl 4-methyl-3-oxo-4-aza-5α-androst-1-ene-17ß-carboxamide |
| 34 | N-(t-butyl) 4-methyl-3-oxo-4-aza-5α-androst-1-ene-17ß-carboxamide |
| 35 | N-octyl 4-methyl-3-oxo-4-aza-5α-androst-1-ene-17ß-carboxamide |
| 36 | N-(2,4,4-trimethyl-2-pentyl) 4-methyl-3-oxo-4-aza-5α-androst-1-ene-17ß-carboxamide |
| 37 | N,N-bis(2-propyl) 4-methyl-3-oxo-4-aza-5α-androst-1-ene-17ß-carboxamide |

## Claims

**Claims for the following Contracting States : AT, BE, CH, LI, DE, FR, GB, IT, LU, NL, SE**

**1.** A process for dehydrogenating a compound of the formula

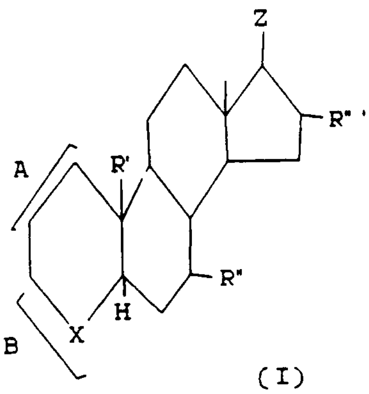

(I)

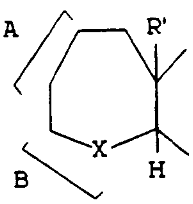

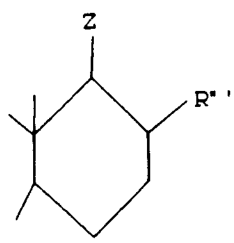

(II) (III)

where Formula (I) may alternatively have the structure of partial Formulas (II) and/or (III); wherein, A is

(1) $-CH_2-CH_2-$;

(2)

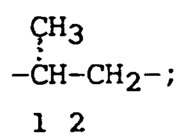

B is

(1)

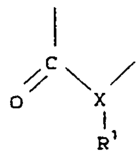

where X is N or O; and

$R^1$ is absent or is

(a) hydrogen;

(b) methyl or ethyl;

(c) $NR^2R^3$ where $R^2$ and $R^3$ are hydrogen or methyl; or

(d) cyano; or

(2)

(a)

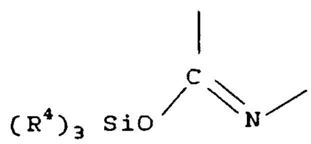

(b)

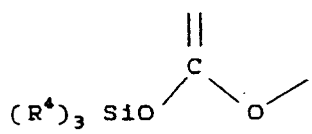

(c)

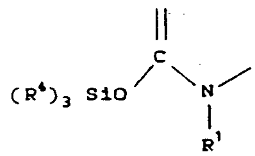

where $R^4$ is any of methyl, $C_{1-8}$ straight or branched chain alkyl, $C_{3-6}$ cycloalkyl, phenyl, or combinations thereof;

R' is hydrogen or methyl;

R'' is hydrogen or $\beta$-methyl;

R''' is hydrogen, $\beta$-methyl or hydroxyl;

Z is

(1) $\beta$-hydrogen and $\alpha$-hydroxyl;

(2) $\alpha$-hydrogen or $\alpha$-hydroxyl and

(a)

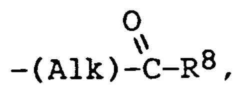

where Alk is present or absent and is a straight or branched hydrocarbon chain of 1 to 12 carbon atoms; and $R^8$ is,

(i) hydrogen,
(ii) hydroxyl,
(iii) $C_{1-12}$ alkyl,
(iv) $NR^9R^{10}$, where $R^9$ and $R^{10}$ are each independently selected from hydrogen; $C_{1-12}$ straight or branched chain alkyl, $C_{1-12}$ straight or branched chain alkyl having a hydrogen substituted with a hydroxy, carboxylic acid or $C_{1-4}$ alkyl ester; $C_{3-6}$ cycloalkyl; phenyl; or $R^9$ and $R^{10}$ taken together with the nitrogen to which they are attached represent a 5-6 membered saturated ring comprising up to one other heteroatom selected from oxygen and nitrogen; or
(v) $OR^{11}$, where $R^{11}$ is M, where M is hydrogen or alkali metal, or $C_{1-18}$ straight or branched chain alkyl; benzyl; or

(b) -(Alk)-$OR^{12}$, where Alk is always present and has the same meaning as above; and $R^{12}$ is

(i) phenyl $C_{1-6}$ alkylcarbonyl,
(ii) $C_{5-10}$ cycloalkylcarbonyl,
(iii) benzoyl, or
(iv) $C_{1-18}$ alkoxycarbonyl;
(v) amino, or $C_{1-8}$ alkyl substituted amino, carbonyl; or
(vi) hydrogen, provided that Alk is a branched $C_3$-$C_8$ chain;

(3)

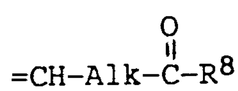

or = CH-Alk-$OR^{12}$, where Alk is present or absent and has the same meaning as above, and $R^8$ and $R^{12}$ have the same meaning as above, and $R^{12}$ is also hydrogen or $C_{1-20}$ alkylcarbonyl;

(4)

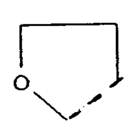

where the dashed bond replaces the 17$\alpha$ hydrogen;

(5) $\alpha$-hydrogen and

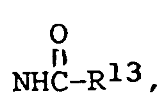

where $R^{13}$ is,

(a) $C_{1-12}$ alkyl; or
(b) $NR^9R^{10}$;

(6) α-hydrogen and cyano; or

(7) α-hydrogen and tetrazolyl; which comprises reacting the compound with a silylating agent in the presence of a quinone to introduce a $\Delta^1$ double bond.

**2.** A process according to Claim 1 wherein the silylating agent is a bistrimethylsilyltrihaloacetamide, bistrimethylsilylacetamide, hexamethyldisilazane, or bistrimethylsilylurea.

**3.** A process according to Claim 2 wherein the quinone is:

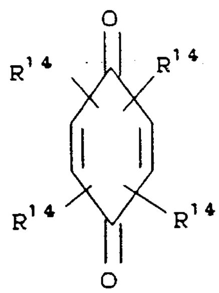

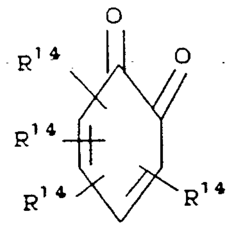

where $R^{14}$ is hydrogen, $C_{1-6}$ alkyl, $C_{1-6}$ alkoxy, halo, nitro or cyano.

**4.** The process according to Claim 3 wherein the quinone is 2,3-dichloro-5,6-dicyano-1,4-benzoquinone.

**5.** A process according to Claim 1 wherein A is $-CH_2-CH_2-$;
B is

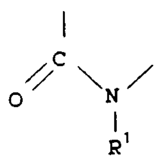

where $R^1$ is
(a) hydrogen;
(b) methyl or ethyl;
R'', R''' are hydrogen; and
Z is

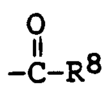

where $R^8$ is
(i) $-NHC_{3-12}$ branched alkyl;
(ii) $-NHC_{3-12}$ branched alkyl having a hydrogen substituted with a hydroxy, carboxylic acid or $C_{1-4}$ alkyl ester;
(iii) $-C_{3-12}$ branched alkyl; or
(iv) $-OCH_3$.

**6.** A process according to Claim 5 wherein the silylating agent is a bistrimethylsilyltrihaloacetamide, bistrimethylsilylacetamide, hexamethyldisilazane, or bistrimethylsilylurea.

**7.** A process according to Claim 6 wherein the silylating agent is bistrimethylsilyltrifluoroacetamide and the quinone is 2,3-dichloro-5,6-dicyano-1,4-benzoquinone.

**8.** A process according to Claim 7 wherein $R^1$ is hydrogen and $R^8$ is -NH-t-butyl.

**9.** A dehydrogenation process which comprises reacting 17β-N-(t-butylcarbamoyl)-4-aza-5α-androstan-3-one with a silylating agent in the presence of a quinone to form 17β-N-(t-butylcarbamoyl)-4-aza-5α-androst-1-en-3-one.

**10.** A process according to Claim 9 wherein the silylating agent is bistrimethylsilyltrifluoroacetamide and the quinone is 2,3-dichloro-5,6-dicyano-1,4-benzoquinone.

**11.** A process according to Claim 1 which comprises reacting 23-methyl-4-aza-21-nor-5α-cholan-3,20-dione with a silyating agent in the presence of a quinone to form the $\Delta^1$ compound.

**12.** A process according to Claim 1 which comprises reacting 22-methyl-4-aza-21-nor-5α-cholan-3,20-dione with a silyating agent in the presence of a quinone to form the $\Delta^1$ compound.

**13.** A process according to Claim 1 which comprises reacting 3-oxo-4-aza-5α-androstan-17β-carboxylic acid with a silyating agent in the presence of a quinone to form the $\Delta^1$ compound.

**14.** A process according to Claim 1 which comprises reacting 17β-(isopropylcarbonyl)-4-aza-5α-androstan-3-one with a silyating agent in the presence of a quinone to form the $\Delta^1$ compound.

**15.** A process according to Claim 1 which comprises reacting 17β-(carbomethoxy)-4-aza-5α-androstan-3-one with a silyating agent in the presence of a quinone to form the $\Delta^1$ compound.

**16.** The compounds of the formula

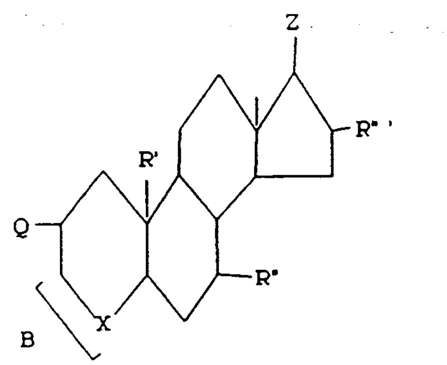

(I)

wherein:
Q is absent or is

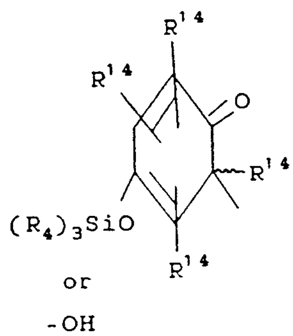

or

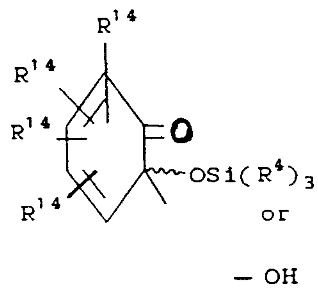

where $R^4$ is methyl, $C_{1-8}$ straight or branched chain alkyl, $C_{3-6}$ cycloalkyl, phenyl, or combinations thereof; and $R^{14}$ is hydrogen, $C_{1-6}$ alkyl, $C_{1-6}$ straight or branched chain alkoxy, halo, nitro or cyano;
B is

(a) when Q is present,

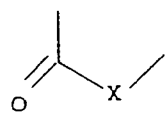

where X is $NR^1$ or O; and $R^1$ is absent or is hydrogen, methyl or ethyl;

(b)

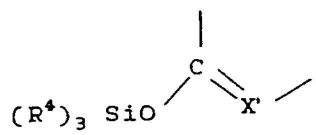

where X' is N;

(c)

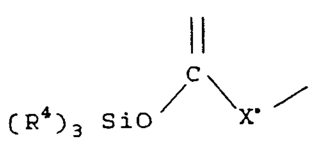

where X'' is O;

Z is

(1) $\beta$-hydrogen and $\alpha$-hydroxyl;

(2) $\alpha$-hydrogen or $\alpha$-hydroxyl and

(a)

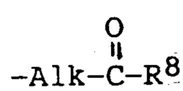,

where Alk is present or absent and is a straight or branched hydrocarbon chain of 1 to 12 carbon atoms; and $R^8$ is,

(i) hydrogen,

(ii) hydroxyl,

(iii) $C_{1-12}$ alkyl

(iv) $NR^9R^{10}$, where $R^9$ and $R^{10}$ are each independently selected from hydrogen; $C_{1-12}$ straight or branched chain alkyl; $C_{1-12}$ straight or branched chain alkyl having a hydrogen substituted with a hydroxy, carboxylic acid or $C_{1-4}$ alkyl ester; $C_{3-6}$ cycloalkyl; phenyl; or $R^9$ and $R^{10}$ taken together with the nitrogen to which they are attached represent a 5-6 membered saturated ring comprising up to one other heteroatom selected from oxygen and nitrogen; or

(v) $OR^{11}$, where $R^{11}$ is M, where M is hydrogen or alkali metal, or $C_{1-18}$ straight or branched chain alkyl; benzyl; or

(b) -(Alk)-$OR^{12}$, where Alk is always present and has the same meaning as above; and $R^{12}$ is

(i) phenyl $C_{1-6}$ alkylcarbonyl,

(ii) $C_{5-10}$ cycloalkylcarbonyl,

(iii) benzoyl, or

(iv) $C_{1-18}$ alkoxycarbonyl;

(v) amino, or $C_{1-8}$ alkyl substituted amino, carbonyl; or

(vi) hydrogen, provided that Alk is a branched $C_3$-$C_8$ chain;

(3)

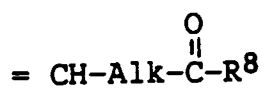

or = CH-Alk-$OR^{12}$, where Alk is present or absent and has the same meaning as above, and $R^8$ and $R^{12}$ have the same meaning as above, and $R^{12}$ is also hydrogen or $C_{1-20}$ alkylcarbonyl;

(4)

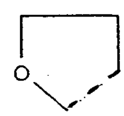

where the dashed bond replaces the 17α hydrogen;

(5) α-hydrogen and

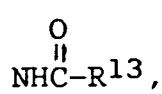

where $R^{13}$ is,

(a) $C_{1-12}$ alkyl; or

(b) $NR^9R^{10}$;

(6) α-hydrogen and cyano; or

(7) α-hydrogen and tetrazolyl;

R' is hydrogen or methyl;

R'' is hydrogen or β-methyl;

R''' is hydrogen, β-methyl or hydroxyl.

**17.** The compounds of Claim 16, wherein

Q is present and $R^4$ is methyl;

B is

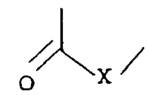

where X is $NR^1$;
R'', R''' are hydrogen;
Z is

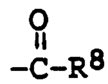

where $R^8$ is
(i) $-NHC_{3-12}$ branched alkyl;
(ii) $-NHC_{3-12}$ branched alkyl having a hydrogen substituted with a hydroxy, carboxylic acid or $C_{1-4}$ alkyl ester;
(iii) $-C_{3-12}$ branched alkyl; or
(iv) $-OCH_3$.

**18.** The compounds of Claim 17 wherein $R^8$ is -NH-t-butyl,

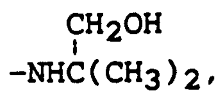

sec-butyl, isobutyl or $-OCH_3$.

**19.** The compound of Claim 18 wherein $R^1$ is hydrogen and $R^8$ is -NH-t-butyl.

**20.** The compound of Claim 19 where Q is

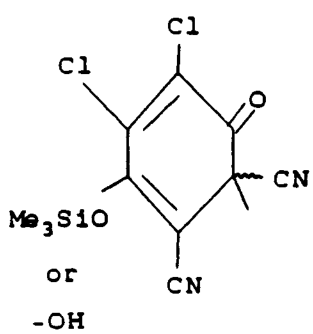

**21.** The compound of Claim 19 where Q is

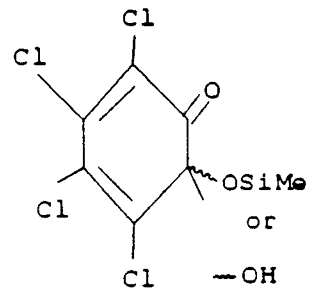

**Claims for the following Contracting States : ES, GR**

**1.** A process for dehydrogenating lactones and lactams, in particular 3-keto-4-azasteroids, of the formula

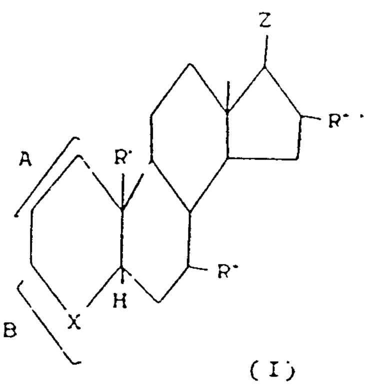

(I)

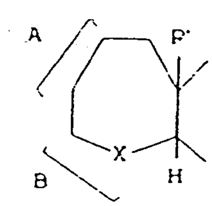

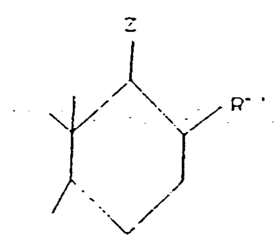

(II) (III)

where Formula (I) may alternatively have the structure of partial Formulas (II) and/or (III); wherein, A is

(1) $-CH_2-CH_2-$;

(2)

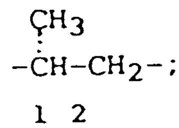

B is

(1)

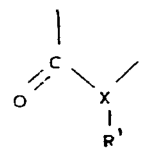

where X is N or O; and

$R^1$ is absent or is

(a) hydrogen;

(b) methyl or ethyl;

(c) $NR^2R^3$ where $R^2$ and $R^3$ are hydrogen or methyl; or

(d) cyano; or

(2)

(a)

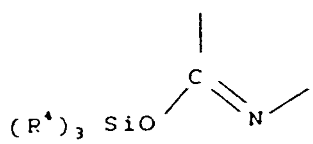

(b)

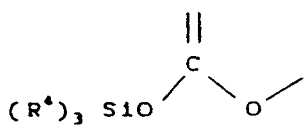

(c)

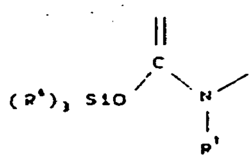

where $R^4$ is any of methyl, $C_{1-8}$ straight or branched chain alkyl, $C_{3-6}$ cycloalkyl, phenyl, or combinations thereof;

R' is hydrogen or methyl;

R'' is hydrogen or $\beta$-methyl;
R''' is hydrogen, $\beta$-methyl or hydroxyl;
Z is
(1) $\beta$-hydrogen and $\alpha$-hydroxyl;
(2) $\alpha$-hydrogen or $\alpha$-hydroxyl and
(a)

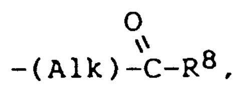

where Alk is present or absent and is a straight or branched hydrocarbon chain of 1 to 12 carbon atoms; and $R^8$ is,
(i) hydrogen,
(ii) hydroxyl,
(iii) $C_{1-12}$ alkyl,
(iv) $NR^9R^{10}$, where $R^9$ and $R^{10}$ are each independently selected from hydrogen; $C_{1-12}$ straight or branched chain alkyl, $C_{1-12}$ straight or branched chain alkyl having a hydrogen substituted with a hydroxy carboxylic acid or $C_{1-4}$ alkyl ester; $C_{3-6}$ cycloalkyl; phenyl; or $R^9$ and $R^{10}$ taken together with the nitrogen to which they are attached represent a 5-6 membered saturated ring comprising up to one other heteroatom selected from oxygen and nitrogen; or
(v) $OR^{11}$, where $R^{11}$ is M, where M is hydrogen or alkali metal, or $C_{1-18}$ straight or branched chain alkyl; benzyl; or
(b) -(Alk)-$OR^{12}$, where Alk is always present and has the same meaning as above; and
$R^{12}$ is
(i) phenyl $C_{1-6}$ alkylcarbonyl,
(ii) $C_{5-10}$ cycloalkylcarbonyl,
(iii) benzoyl, or
(iv) $C_{1-18}$ alkoxycarbonyl;
(v) amino, or $C_{1-8}$ alkyl substituted amino, carbonyl; or
(vi) hydrogen, provided that Alk is a branched $C_3$-$C_8$ chain;
(3)

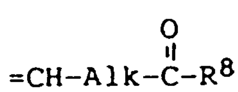

or =CH-Alk-$OR^{12}$ where Alk is present or absent and has the same meaning as above, and $R^8$ and $R^{12}$ have the same meaning as above, and $R^{12}$ is also hydrogen or $C_{1-20}$ alkylcarbonyl;
(4)

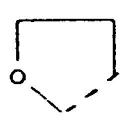

where the dashed bond replaces the 17$\alpha$ hydrogen;
(5) $\alpha$-hydrogen and

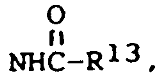

where $R^{13}$ is,

(a) $C_{1-12}$ alkyl; or
(b) $NR^9R^{10}$
(6) α-hydrogen and cyano; or
(7) α-hydrogen and tetrazolyl; which comprises reacting the compound with a silylating agent in the presence of a quinone in order to introduce a $\Delta^1$ double bond.

**2.** A process according to Claim 1 wherein the silylating agent is a bistrimethylsilyltrihaloacetamide, bistrimethylsilylacetamide, hexamethyldisilazane, or bistrimethylsilylurea.

**3.** A process according to Claim 2 wherein the quinone is:

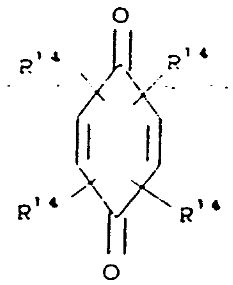

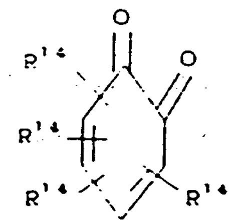

where $R^{14}$ is hydrogen, $C_{1-6}$alkyl, $c_{1-6}$alkoxy, halo, nitro or cyano.

**4.** The process according to Claim 3 wherein the quinone is 2,3-dichloro-5,6-dicyano-1,4-benzoquinone.

**5.** A process according to Claim 1 wherein
A is $-CH_2-CH_2-$;
B is

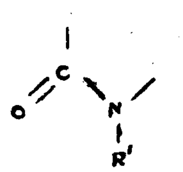

where $R^1$ is
(a) hydrogen;
(b) methyl or ethyl;
R'', R''' are hydrogen; and
Z is

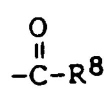

where $R^8$ is
(i) $-NHC_{3-12}$ branched alkyl;
(ii) $-NHC_{3-12}$ branched alkyl having a hydrogen substituted with a hydroxy, carboxylic acid or $C_{1-4}$ alkyl ester;
(iii) $-C_{3-12}$ branched alkyl; or

(iv) $-OCH_3$.

6. A process according to Claim 5 wherein the silylating agent is a bistrimethylsilyltrihaloacetamide, bistrimethylsilylacetamide, hexamethyldisilazane, or bistrimethylsilylurea.

7. A process according to Claim 6 wherein the silylating agent is bistrimethylsilyltrifluoroacetamide and the quinone is 2,3-dichloro-5,6-dicyano-1,4-benzoquinone.

8. A process according to Claim 7 wherein $R^1$ is hydrogen and $R^8$ is -NH-t-butyl.

9. A dehydrogenation process according to Claim 1, which comprises reacting 17β-N-(t-butylcarbamoyl)-4-aza-5α-androstan-3-one with a silylating agent in the presence of a quinone to form 17β-N-(t-butylcarbamoyl)-4-aza-5α-androst-1-en-3-one.

10. A process according to Claim 9 wherein the silylating agent is bistrimethylsilyltrifluoroacetamide and the quinone is 2,3-dichloro-5,6-dicyano-1,4-benzoquinone.

11. A process according to Claim 1 which comprises reacting 23-methyl-4-aza-21-nor-5α-cholan-3,20-dione with a silylating agent in the presence of a quinone to form the $\Delta^1$ compound.

12. A process according to Claim 1 which comprises reacting 22-methyl-4-aza-21-nor-5α-cholan-3,20-dione with a silylating agent in the presence of a quinone to form the $\Delta^1$ compound.

13. A process according to Claim 1 which comprises reacting 3-oxo-4-aza-5α-androstan-17β-carboxylic acid with a silylating agent in the presence of a quinone to form the $\Delta^1$ compound.

14. A process according to Claim 1 which comprises reacting 17β-(isopropylcarbonyl)-4-aza-5α-androstan-3-one with a silylating agent in the presence of a quinone to form the $\Delta^1$ compound.

15. A process according to Claim 1 which comprises reacting 17β-(carbomethoxy)-4-aza-5α-androstan-3-one with a silylating agent in the presence of a quinone to form the $\Delta^1$ compound.

16. A process for the preparation of compounds having the following formula:

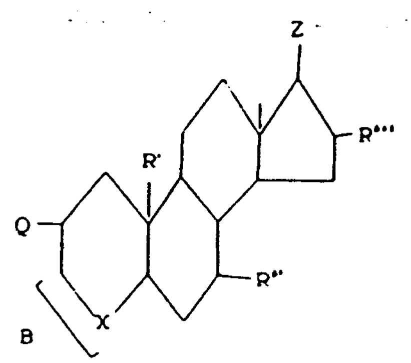

(I)

wherein:
Q is absent or is

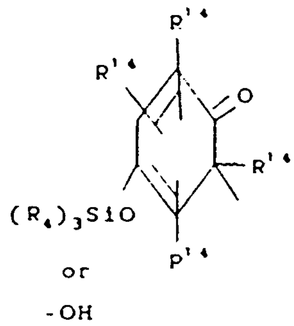

or

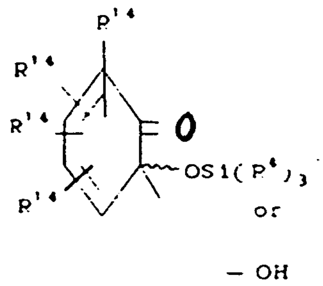

where $R^4$ is methyl, $C_{1-8}$ straight or branched chain alkyl, $C_{3-6}$ cycloalkyl, phenyl, or combinations thereof; and $R^{14}$ is hydrogen, $C_{1-6}$ alkyl, $C_{1-6}$ straight or branched chain alkoxy, halo, nitro or cyano;
B is

(a) when Q is present,

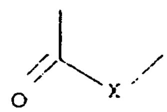

where X is $NR^1$ or O; and $R^1$ is absent or is hydrogen, methyl or ethyl;

(b)

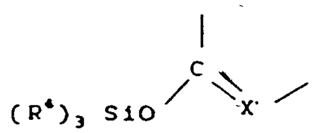

where X' is N;

(c)

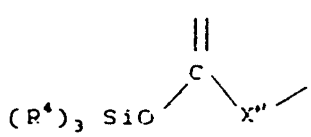

where X'' is O;

Z is

(1) $\beta$-hydrogen and $\alpha$-hydroxyl;

(2) $\alpha$-hydrogen or $\alpha$-hydroxyl and

(a)

$$-\mathrm{Alk}-\overset{\mathrm{O}}{\overset{\|}{\mathrm{C}}}-R^8,$$

where Alk is present or absent and is a straight or branched hydrocarbon chain of 1 to 12 carbon atoms; and $R^8$ is,

(i) hydrogen,
(ii) hydroxyl,
(iii) $C_{1-12}$ alkyl
(iv) $NR^9R^{10}$, where $R^9$ and $R^{10}$ are each independently selected from hydrogen; $C_{1-12}$ straight or branched chain alkyl; $C_{1-12}$ straight or branched chain alkyl having a hydrogen substituted with a hydroxy, carboxylic acid or $C_{1-4}$ alkyl ester; $C_{3-6}$ cycloalkyl; phenyl; or $R^9$ and $R^{10}$ taken together with the nitrogen to which they are attached represent a 5-6 membered saturated ring comprising up to one other heteroatom selected from oxygen and nitrogen; or
(v) $OR^{11}$, where $R^{11}$ is M, where M is hydrogen or alkali metal, or $C_{1-18}$ straight or branched chain alkyl; benzyl; or

(b) -(Alk)-$OR^{12}$, where Alk is always present and has the same meaning as above; and $R^{12}$ is
(i) phenyl $C_{1-6}$ alkylcarbonyl,
(ii) $C_{5-10}$ cycloalkylcarbonyl,
(iii) benzoyl, or
(iv) $C_{1-18}$ alkoxycarbonyl;
(v) amino, or $C_{1-8}$ alkyl substituted amino, carbonyl; or
(vi) hydrogen, provided that Alk is a branched $C_3$-$C_8$ chain;

(3)

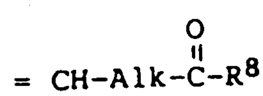

or = CH-Alk-$OR^{12}$, where Alk is present or absent and has the same meaning as above, and $R^8$ and $R^{12}$ have the same meaning as above, and $R^{12}$ is also hydrogen or $C_{1-20}$ alkylcarbonyl;

(4)

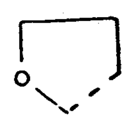

where the dashed bond replaces the 17$\alpha$ hydrogen;

(5) $\alpha$-hydrogen and

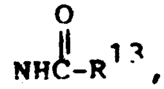

where $R^{13}$ is,
(a) $C_{1-12}$ alkyl; or
(b) $NR^9R^{10}$;

(6) $\alpha$-hydrogen and cyano; or
(7) $\alpha$-hydrogen and tetrazolyl;
R' is hydrogen or methyl;
R'' is hydrogen or $\beta$-methyl;
R''' is hydrogen, $\beta$-methyl or hydroxyl,

characterized by reacting a lactone or lactam, in particular a 3-keto-4-azasteroid, of the formula

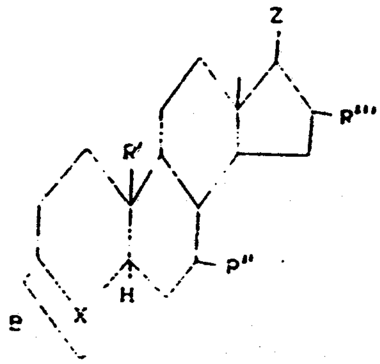

wherein B, Z, X, R'; R" and R'" are those defined above, with a silylating agent in the presence of a quinone.

**17.** A process according to Claim 16 wherein the silylating agent is a bistrimethylsilyltrihaloacetamide, bistrimethylsilylacetamide, hexamethyldisilazane, or bistrimethylsilylurea.

**18.** A process according to Claim 17 wherein the quinone is:

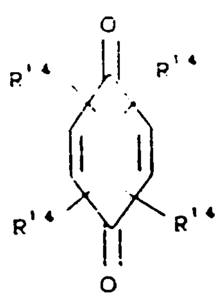

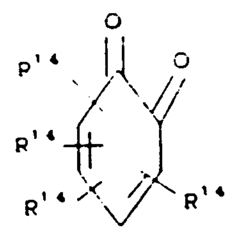

where $R^{14}$ is hydrogen, $C_{1-6}$alkyl, $C_{1-6}$alkoxy, halo, nitro or cyano.

**19.** A process according to Claim 18 wherein the quinone is 2,3-dichloro-5,6-dicyano-1,4-benzoquinone.

**20.** A process according to Claim 16 wherein
Q is present and $R^4$ is methyl;
B is

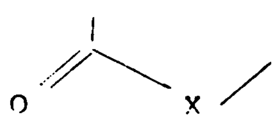

where X is $NR^1$;
R", R'" are hydrogen;
Z is

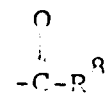

where $R^8$ is

(i) $-NHC_{3-12}$ branched alkyl;

(ii) $-NHC_{3-12}$ branched alkyl having a hydrogen substituted with a hydroxy, carboxylic acid or $C_{1-4}$ alkyl ester;

(iii) $-C_{3-12}$ branched alkyl; or

(iv) $-OCH_3$.

**21.** A process according to Claim 20 wherein $R^8$ is -NH-t-butyl,

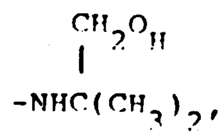

sec-butyl, isobutyl or $-OCH_3$.

**22.** A process according to Claim 21 wherein $R^1$ is hydrogen and $R^8$ is -NH-t-butyl.

**23.** A process according to Claim 22 where O is

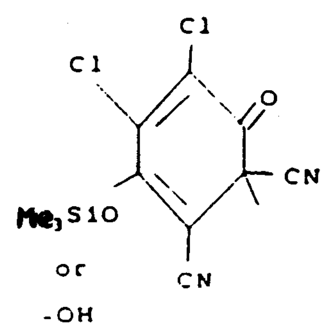

**24.** A process according to Claim 22 where O is

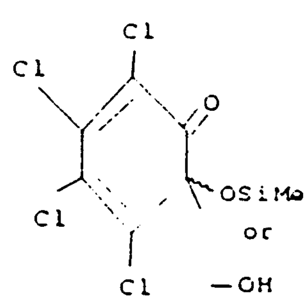

## Patentansprüche

### Patentansprüche für folgende Vertragsstaaten : AT, BE, CH, LI, DE, FR, GB, IT, LU, NL, SE

**1.** Verfahren zur Dehydrierung einer Verbindung der Formel:

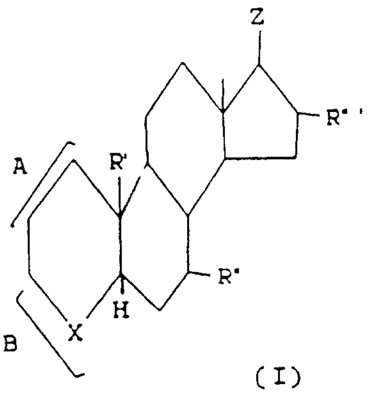

(I)

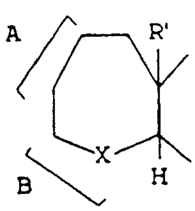

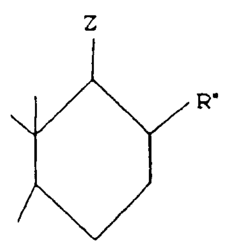

(II) (III)

worin Formel (I) alternativ die Struktur der Teilformeln (II) und/oder (III) besitzen kann, worin A für folgendes steht:

(1) $-CH_2-CH_2-$

(2)

$$\begin{array}{c} CH_3 \\ \vdots \\ -CH-CH_2-; \\ 1\;\;2 \end{array}$$

B für folgendes steht:

(1)

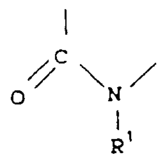

worin

X N oder O bedeutet und

$R^1$ fehlt oder für

(a) Wasserstoff,

(b) Methyl oder Ethyl,

(c) $NR^2R^3$, worin $R^2$ und $R^3$ Wasserstoff oder Methyl bedeuten, oder

(d) Cyano steht oder

(2)

(a)

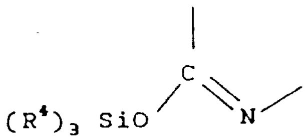

(b)

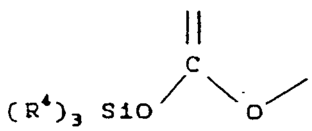

(c)

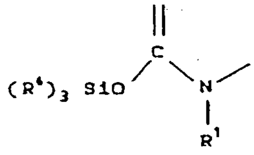

worin $R^4$ eines von Methyl, geradkettigem oder verzweigtem $C_{1-8}$-Alkyl, $C_{3-6}$-Cycloalkyl, Phenyl oder für Kombinationen davon steht,

R' Wasserstoff oder Methyl bedeutet

R'' Wasserstoff oder $\beta$-Methyl bedeutet,
R''' Wasserstoff, $\beta$-Methyl oder Hydroxy bedeutet,

Z für folgendes stellt:

(1) $\beta$-Wasserstoff und $\alpha$-Hydroxy,

(2) $\alpha$-Wasserstoff oder $\alpha$-Hydroxy und

(a)

$$-(Alk)-\overset{O}{\overset{\|}{C}}-R^8,$$

worin Alk vorhanden ist oder fehlt und für eine geradkettige oder verzweigte Kohlenwasserstoffkette mit 1 bis 12 Kohlenstoffatomen steht und $R^8$ für folgendes steht:

(i) Wasserstoff,

(ii) Hydroxy,

(iii) $C_{1-12}$-Alkyl,

(iv) $NR^9R^{10}$, worin $R^9$ und $R^{10}$ jeweils unabhängig ausgewählt werden aus Wasserstoff, geradkettigem oder verzweigtem $C_{1-12}$-Alkyl, geradkettigem oder verzweigtem $C_{1-12}$-Alkyl, bei dem ein Wasserstoff durch Hydroxy, Carbonsäure oder $C_{1-4}$-Alkylester ersetzt ist, $C_{3-6}$-Cycloalkyl, Phenyl oder worin $R^9$ und $R^{10}$ zusammengenommen mit dem Stickstoffatom, an das sie angeknüpft sind, einen 5-6gliedrigen gesättigten Ring darstellen, der bis zu einem weiteren Heteroatom umfaßt, ausgewählt aus Sauerstoff und Stickstoff, oder

(v) $OR^{11}$, worin $R^{11}$ für M, worin M Wasserstoff oder Alkalimetall bedeutet, oder geradkettiges oder verzweigtes $C_{1-18}$-Alkyl, Benzyl steht, oder

(b) -(Alk)-$OR^{12}$, worin Alk immer vorhanden ist und die gleiche Bedeutung wie oben besitzt und $R^{12}$ für folgendes steht:

(i) Phenyl-$C_{1-6}$-alkylcarbonyl,

(ii) $C_{5-10}$-Cycloalkylcarbonyl,

(iii) Benzoyl oder

(iv) $C_{1-18}$-Alkoxycarbonyl,

(v) Amino oder $C_{1-8}$-Alkyl-substituiertes Amino, Carbonyl oder

(vi) Wasserstoff, mit der Maßgabe, daß Alk eine verzweigte $C_3$-$C_8$-Kette darstellt,

(3)

$$=CH-Alk-\overset{O}{\overset{\|}{C}}-R^8$$

oder $=CH-Alk-OR^{12}$, worin Alk vorhanden ist oder fehlt und dieselbe Bedeutung wie vorstehend besitzt und $R^8$ und $R^{12}$ dieselben Bedeutungen wie oben besitzen und $R^{12}$ ferner für Wasserstoff oder $C_{1-20}$-Alkylcarbonyl steht,

(4)

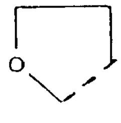

worin die gestrichelte Bindung den 17$\alpha$-Wasserstoff ersetzt,

(5) α-Wasserstoff und

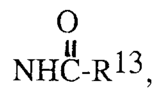

worin $R^{13}$ für
(a) $C_{1-12}$-Alkyl oder
(b) $NR^9R^{10}$ steht
(6) α-Wasserstoff und Cyano oder
(7) α-Wasserstoff und Tetrazolyl,
welches die Umsetzung der Verbindung mit einem Silylierungsmittel in Gegenwart eines Chinons zur Einführung einer $\Delta^1$-Doppelbindung umfaßt.

**2.** Verfahren nach Anspruch 1, bei dem das Silylierungsmittel Bistrimethylsilyltrihalogenacetamid, Bistrimethylsilylacetamid, Hexamethyldisilazan oder Bistrimethylsilylharnstoff ist.

**3.** Verfahren nach Anspruch 2, bei dein das Chinon

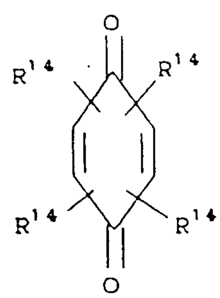

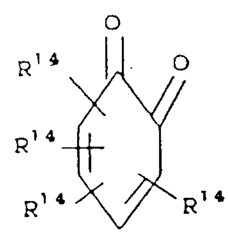

ist, worin $R^{14}$ für Wasserstoff, $C_{1-6}$-Alkyl, $C_{1-6}$-Alkoxy, Halogen, Nitro oder Cyano steht.

**4.** Verfahren nach Anspruch 3, bei dein das Chinon 2,3-Dichlor-5,6-dicyano-1,4-benzochinon ist.

**5.** Verfahren nach Anspruch 1, bei dem
A für $-CH_2CH_2-$ steht,
B für

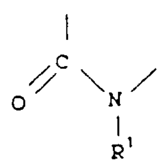

steht, worin
$R^1$ für
(a) Wasserstoff,
(b) Methyl oder Ethyl steht,
R'', R''' für Wasserstoff stehen und

$$Z \text{ für } -\overset{\displaystyle O}{\overset{\|}{C}}-R^8$$

steht, worin $R^8$ für

(i) verzweigtes $C_{3-12}$-Alkyl-NH,

(ii) verzweigtes $C_{3-12}$-Alkyl-NH, bei dem ein Wasserstoff durch Hydroxy, Carbonsäure oder $C_{1-4}$-Alkylester substituiert ist,

(iii) verzweigtes $C_{3-12}$-Alkyl oder

(iv) $-OCH_3$ steht.

**6.** Verfahren nach Anspruch 5, bei dem das Silylierungsmittel Bistrimethylsilyltrihalogenacetamid, Bistrimethylsilylacetamid, Hexamethyldisilazan oder Bistrimethylsilylharnstoff ist.

**7.** Verfahren nach Anspruch 6, bei dem das Silylierungsmittel Bistrimethylsilyltrifluoracetamid ist und das Chinon 2,3-Dichlor-5,6-dicyano-1,4-benzochinon ist.

**8.** Verfahren nach Anspruch 7, bei dem $R^1$ für Wasserstoff steht und $R^8$-NH-t-Butyl bedeutet.

**9.** Dehydrierungsverfahren, das die Umsetzung von 17$\beta$-N-(t-Butylcarbamoyl)-4-aza-5$\alpha$-androstan-3-on mit einem Silylierungsmittel in Gegenwart eines Chinons unter Bildung von 17$\beta$-N-(t-Butylcarbamoyl)-4-aza-5$\alpha$-androst-1-en-3-on umfaßt.

**10.** Verfahren nach Anspruch 9, bei dem das Silylierungsmittel Bistrimethylsilyltrifluoracetamid ist und das Chinon 2,3-Dichlor-5,6-dicyano-1,4-benzochinon ist.

**11.** Verfahren nach Anspruch 1, welches die Umsetzung von 23-Methyl-4-aza-21-nor-5 $\alpha$-cholan-3,20-dion mit einem Silylierungsmittel in Gegenwart eines Chinons unter Bildung der $\Delta^1$-Verbindung umfaßt.

**12.** Verfahren nach Anspruch 1, das die Umsetzung von 22-Methyl-4-aza-21-nor-5$\alpha$-cholan-3,20-dion mit einem Silylierungsmittel in Gegenwart eines Chinons unter Bildung der $\Delta^1$-Verbindung umfaßt.

**13.** Verfahren nach Anspruch 1, welches die Umsetzung von 3-Oxo-4-aza-5$\alpha$-androstan-17$\beta$-carbonsäure mit einem Silylierungsmittel in Gegenwart eines Chinons unter Bildung der $\Delta^1$-Verbindung umfaßt.

**14.** Verfahren nach Anspruch 1, das die Umsetzung von 17$\beta$-(Isopropylcarbonyl)-4-aza-5$\alpha$-androstan-3-on mit einem Silylierungsmittel in Gegenwart eines Chinons unter Bildung der $\Delta^1$-Verbindung umfaßt.

**15.** Verfahren nach Anspruch 1, das die Umsetzung von 17$\beta$-(Carbomethoxy)-4-aza-5$\alpha$-androstan-3-on mit einem Silylierungsmittel in Gegenwart eines Chinons unter Bildung der $\Delta^1$-Verbindung umfaßt.

**16.** Verbindungen der Formel

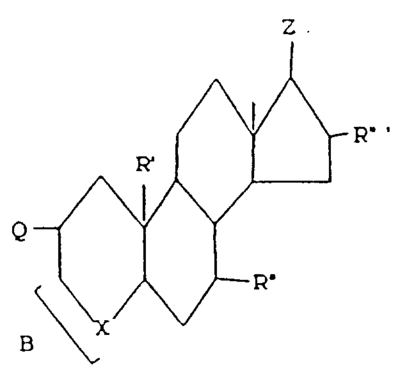

(I)

worin:
Q fehlt oder für

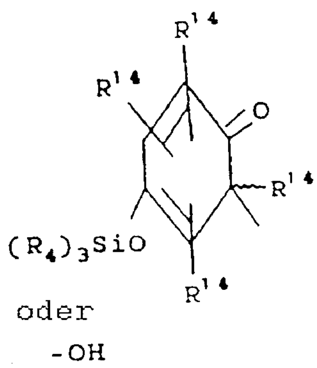

oder

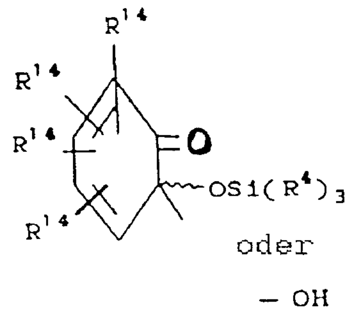

steht, worin

$R^4$ für Methyl, geradkettiges oder verzweigtes $C_{1-8}$-Alkyl, $C_{3-6}$-Cycloalkyl, Phenyl oder für Kombinationen davon steht und $R^{14}$ für Wasserstoff, $C_{1-6}$-Alkyl, geradkettiges oder verzweigtes $C_{1-6}$-Alkoxy, Halogen, Nitro oder Cyano steht,

B für folgendes stellt:

(a)

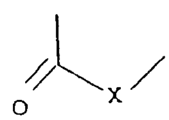

wenn Q vorhanden ist

Worin X für $NR^1$ oder O stellt und $R^1$ fehlt oder für Wasserstoff, Methyl oder Ethyl steht,

(b)

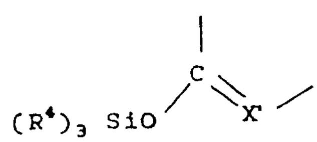

worin X' N bedeutet,

(c)

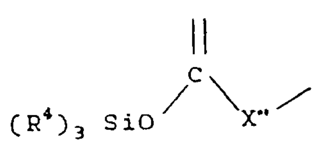

worin X" O bedeutet,

Z für folgendes steht:

(1) $\beta$-Wasserstoff und $\alpha$-Hydroxy,

(2) $\alpha$-Wasserstoff oder $\alpha$-Hydroxy und

(a)

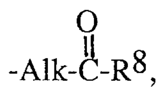

worin Alk vorhanden ist oder fehlt und eine geradkettige oder verzweigte Kohlenwasserstofflette mit 1 bis 12 Kohlenstoffatomen ist und $R^8$ für folgendes steht:

(i) Wasserstoff,

(ii) Hydroxy,

(iii) $C_{1-12}$-Alkyl,

(iv) $NR^9R^{10}$, worin $R^9$ und $R^{10}$ jeweils unabhängig ausgewählt werden aus Wasserstoff, geradkettigem oder verzweigtem $C_{1-12}$-Alkyl, geradkettigem oder verzweigtem $C_{1-12}$-Alkyl, bei dein ein Wasserstoff durch Hydroxy, Carbonsäure oder $C_{1-4}$-Alkylester substituiert ist, $C_{3-6}$-Cycloalkyl, Phenyl, oder $R^9$ und $R^{10}$ zusammengenommen mit dem Stickstoffatom, an das sie angeknüpft sind, einen 5- bis 6-gliedrigen gesättigten Ring darstellen, der bis zu einem weiteren Heteroatom umfaßt, ausgewählt aus Sauerstoff und Stickstoff, oder

(v) $OR^{11}$, worin $R^{11}$ für M steht, worin M Wasserstoff oder Alkalimetall bedeutet, oder geradkettiges oder verzweigtes $C_{1-18}$-Alkyl, Benzyl oder

(b) -(Alk)-$OR^{12}$, worin Alk immer vorhanden ist und die gleiche Bedeutung wie oben besitzt und $R^{12}$ für

(i) Phenyl-$C_{1-6}$-alkylcarbonyl,

(ii) $C_{5-10}$-Cycloalkylcarbonyl,

(iii) Benzoyl oder

(iv) $C_{1-18}$-Alkoxycarbonyl,

(v) Amino oder $C_{1-8}$-Alkyl, substituiertes Amino, Carbonyl oder

(vi) Wasserstoff steht, mit der Maßgabe, daß Alk eine verzweigte $C_3$-$C_8$-Kette bedeutet,

(3)

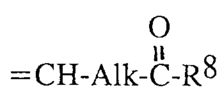

oder =CH-Alk-$OR^{12}$, worin Alk vorhanden ist oder fehlt und dieselbe Bedeutung wie oben besitzt und $R^8$ und $R^{12}$ dieselbe Bedeutung wie oben besitzen und $R^{12}$ ferner für Wasserstoff oder $C_{1-20}$-Alkylcarbonylsteht,

(4)

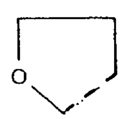

worin die gestrichelte Bindung den 17α-Wasserstoff ersetzt,

(5) α-Wasserstoff und

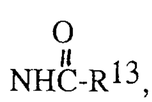

worin $R^{13}$ für

(a) $C_{1-12}$-Alkyl oder
(b) $NR^9R^{10}$ steht,
(6) α-Wasserstoff und Cyano oder
(7) α-Wasserstoff und Tetrazolyl,
R' Wasserstoff und Methyl bedeutet,
R'' Wasserstoff oder β-Methyl bedeutet,
R''' Wasserstoff, β-Methyl oder Hydroxy bedeutet.

**17.** Verbindung nach Anspruch 16, worin
Q vorhanden ist und $R^4$ Methyl bedeutet,
B für

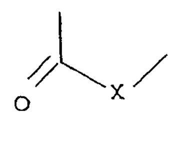

steht, worin X $NR^1$ bedeutet,
R'', R''' für Wasserstoff stehen, Z für

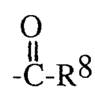

steht, worin $R^8$ für folgendes steht:
(i) verzweigtes $C_{3-12}$-Alkyl-NH,
(ii) verzweigtes $C_{3-12}$-Alkyl-NH, bei dem ein Wasserstoff mit Hydroxy, Carbonsäure oder $C_{1-4}$-Alkylester substituiert ist,
(iii) verzweigtes -$C_{3-12}$-Alkyl oder
(iv) -$OCH_3$.

**18.** Verbindungen nach Anspruch 17, worin
$R^8$ für -NH-t-Butyl,

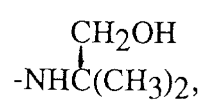

sec-Butyl, Isobutyl oder -$OCH_3$ steht.

**19.** Verbindung nach Anspruch 18, worin $R^1$ für Wasserstoff steht und $R^8$ -NH-t-Butyl bedeutet.

**20.** Verbindung nach Anspruch 19, worin Q für

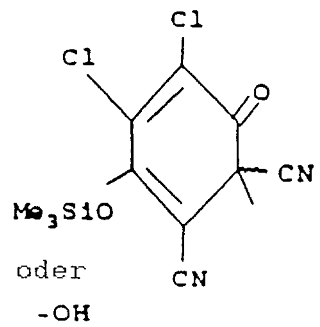

steht.

**21.** Verbindung nach Anspruch 19, worin Q für

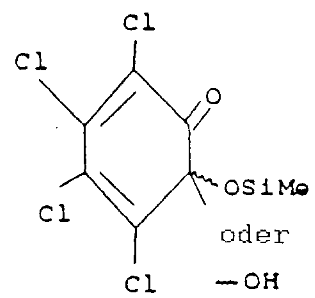

steht.

**Patentansprüche für folgende Vertragsstaaten : ES, GR**

**1.** Verfahren zur Dehydrierung von Lactonen und Lactamen, insbesondere von 3-Keto-4-azasteroiden der Formel:

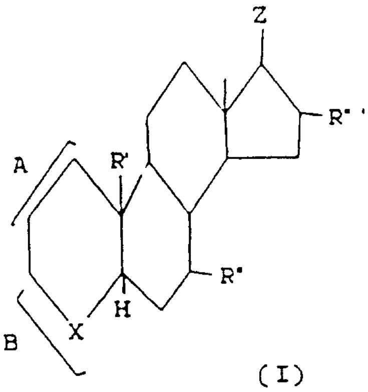

(I)

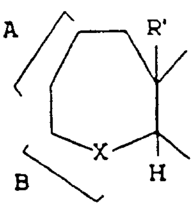

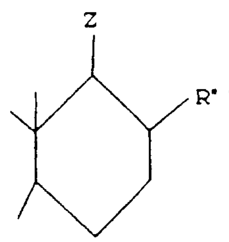

(II) (III)

worin Formel (I) alternativ die Struktur der Teilformeln (II) und/oder (III) besitzen kann, worin A für folgendes steht:

(1) $-CH_2-CH_2-$

(2)

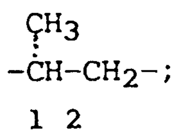

B für folgendes steht:

(1)

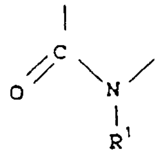

worin

X N oder O bedeutet und

$R^1$ fehlt oder für

(a) Wasserstoff,

(b) Methyl oder Ethyl,
(c) $NR^2R^3$, worin $R^2$ und $R^3$ Wasserstoff oder Methyl bedeuten oder
(d) Cyano steht, oder
(2)
(a)

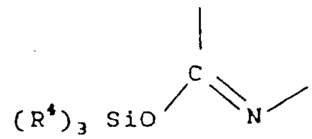

(b)

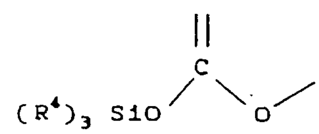

(c)

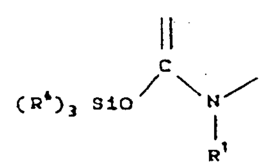

worin $R^4$ eines von Methyl, geradkettigem oder verzweigtem $C_{1-8}$-Alkyl, $C_{3-6}$-Cycloalkyl, Phenyl oder für Kombinationen davon steht,

R' Wasserstoff oder Methyl bedeutet
R'' Wasserstoff oder $\beta$-Methyl bedeutet,
R''' Wasserstoff, $\beta$-Methyl oder Hydroxy bedeutet,

Z für folgendes steht:
(1) $\beta$-Wasserstoff und $\alpha$-Hydroxy,
(2) $\alpha$-Wasserstoff oder $\alpha$-Hydroxy und
(a)

$$-(Alk)-\overset{O}{\overset{\|}{C}}-R^{8},$$

worin Alk vorhanden ist oder fehlt und für eine geradkettige oder verzweigte Kohlenwasserstoffkette mit 1 bis 12 Kohlenstoffatomen steht und $R^8$ für folgendes steht:
(i) Wasserstoff,
(ii) Hydroxy,
(iii) $C_{1-12}$-Alkyl,
(iv) $NR^9R^{10}$, worin $R^9$ und $R^{10}$ jeweils unabhängig ausgewählt werden aus Wasserstoff, geradkettigem oder verzweigtem $C_{1-12}$-Alkyl, geradkettigem oder verzweigtem $C_{1-12}$-Alkyl, bei dem ein Wasserstoff durch Hydroxy, Carbonsäure oder $C_{1-4}$-Alkylester ersetzt ist, $C_{3-6}$-

Cycloalkyl, Phenyl oder worin $R^9$ und $R^{10}$ zusammengenommen mit dem Stickstoff, an den sie angeknüpft sind, einen 5-6gliedrigen gesättigten, Ring darstellen, der bis zu einem weiteren Heteroatom umfaßt, ausgewählt aus Sauerstoff und Stickstoff, oder

(v) $OR^{11}$, worin $R^{11}$ für M, worin M Wasserstoff oder Alkalimetall bedeutet, oder geradkettiges oder verzweigtes $C_{1-18}$-Alkyl, Benzyl, oder

(b) -(Alk)-$OR^{12}$, worin Alk immer vorhanden ist und die gleiche Bedeutung wie oben besitzt und $R^{12}$ für folgendes steht:

(i) Phenyl-$C_{1-6}$-alkylcarbonyl,
(ii) $C_{5-10}$-Cycloalkylcarbonyl,
(iii) Benzoyl oder
(iv) $C_{1-18}$-Alkoxycarbonyl,
(v) Amino oder $C_{1-8}$-Alkyl-substituiertes Amino, Carbonyl oder
(vi) Wasserstoff, mit der Maßgabe, daß Alk eine verzweigte $C_3$-$C_8$-Kette darstellt,

(3)

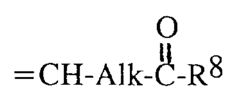

oder =CH-Alk-$OR^{12}$, worin Alk vorhanden ist oder fehlt und dieselbe Bedeutung wie vorstehend besitzt und $R^8$ und $R^{12}$ dieselben Bedeutungen wie oben besitzen und $R^{12}$ ferner für Wasserstoff oder $C_{1-20}$-Alkylcarbonyl steht,

(4)

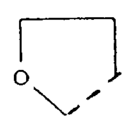

worin die gestrichelte Bindung den 17α-Wasserstoff ersetzt,

(5) a-Wasserstoff und

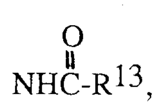

worin $R^{13}$ für
(a) $C_{1-12}$-Alkyl oder
(b) $NR^9R^{10}$ steht,

(6) α-Wasserstoff und Cyano oder

(7) α-Wasserstoff und Tetrazolyl, welches die Umsetzung der Verbindung mit einem Silylierungsmittel in Gegenwart eines Chinons zur Einführung einer $\Delta^1$-Doppelbindung umfaßt.

**2.** Verfahren nach Anspruch 1, bei dem das Silylierungsmittel Bistrimethylsilyltrihalogenacetamid, Bistrimethylsilylacetamid, Hexamethyldisilazan oder Bistrimethylsilylharnstoff ist.

**3.** Verfahren nach Anspruch 2, bei dem das Chinon

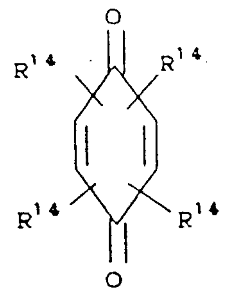 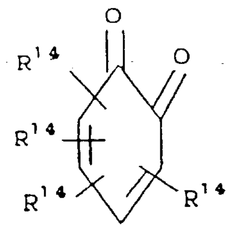

ist, worin $R^{14}$ für Wasserstoff, $C_{1-6}$-Alkyl, $C_{1-6}$-Alkoxy, Halogen, Nitro oder Cyano steht.

**4.** Verfahren nach Anspruch 3, bei dem das Chinon 2,3-Dichlor-5,6-dicyano-1,4-benzochinon ist.

**5.** Verfahren nach Anspruch 1, bei dem
A für $-CH_2-CH_2-$ steht,
B für

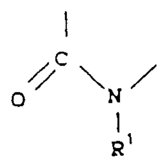

steht, worin
$R^1$ für
(a) Wasserstoff,
(b) Methyl oder Ethyl steht,
R'', R''' für Wasserstoff stehen und
Z für

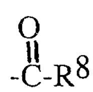

steht, worin $R^8$ für
(i) verzweigtes $C_{3-12}$-Alkyl-NH,
(ii) verzweigtes $C_{3-12}$-Alkyl-NH, bei dem ein Wasserstoff durch Hydroxy, Carbonsäure oder $C_{1-4}$-Alkylester substituiert ist,
(iii) verzweigtes $-C_{3-12}$-Alkyl oder
(iv) $-OCH_3$ steht.

**6.** Verfahren nach Anspruch 5, bei dem das Silylierungsmittel Bistrimethylsilyltrihalogenacetamid, Bistrimethylsilylacetamid, Hexamethyldisilazan oder Bistrimethylsilylharnstoff ist.

**7.** Verfahren nach Anspruch 6, bei dem das Silylierungsmittel Bistrimethylsilyltrifluoracetamid ist und das Chinon 2,3-Dichlor-5,6-dicyano-1,4-benzochinon ist.

8. Verfahren nach Anspruch 7, bei dem $R^1$ für Wasserstoff steht und $R^8$-NH-t-Butyl bedeutet.

9. Dehydrierungsverfahren, das die Umsetzung von 17β-N-(t-Butylcarbamoyl)-4-aza-5α-androstan-3-on mit einem Silylierungsmittel in Gegenwart eines Chinons unter Bildung von 17β-N-(t-Butylcarbamoyl)-4-aza-5α-androst-1-en-3-on umfaßt.

10. Verfahren nach Anspruch 9, bei dem das Silylierungsmittel Bistrimethylsilyltrifluoracetamid ist und das Chinon 2,3-Dichlor-5,6-dicyano-1,4-benzochinon ist.

11. Verfahren nach Anspruch 1, welches die Umsetzung von 23-Methyl-4-aza-21-nor-5 α-cholan-3,20-dion mit einem Silylierungsmittel in Gegenwart eines Chinons unter Bildung der $\Delta^1$-Verbindung umfaßt.

12. Verfahren nach Anspruch 1, das die Umsetzung von 22-Methyl-4-aza-21-nor-5α-cholan-3,20-dion mit einem Silylierungsmittel in Gegenwart eines Chinons unter Bildung der $\Delta^1$-Verbindung umfäßt.

13. Verfahren nach Anspruch 1, welches die Umsetzung von 3-Oxo-4-aza-5α-androstan-17β-carbonsäure mit einem Silylierungsmittel in Gegenwart eines Chinons unter Bildung der $\Delta^1$-Verbindung unfaßt.

14. Verfahren nach Anspruch 1, das die Umsetzung von 17β-(Isopropylcarbonyl)-4-aza-5α-androstan-3-on mit einem Silylierungsmittel in Gegenwart eines Chinons unter Bildung der $\Delta^1$-Verbindung umfaßt.

15. Verfahren nach Anspruch 1, das die Umsetzung von 17β-(Carbomethoxy)-4-aza-5α-androstan-3-on mit einen, Silylierungsmittel in Gegenwart eines Chinons unter Bildung der $\Delta^1$-Verbindung umfaßt.

16. Verfahren zu Herstellung von Verbindungen der Formel

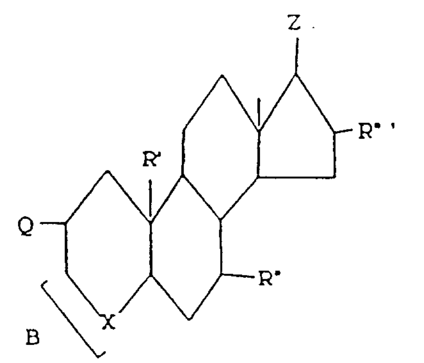

(I)

worin
Q fehlt oder für

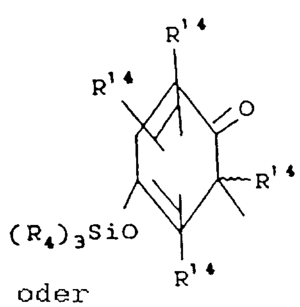

oder
-OH

oder

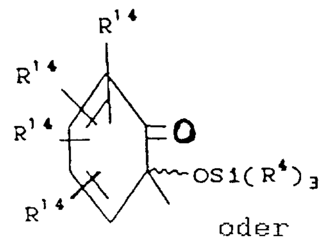

oder
– OH

steht, worin

$R^4$ für Methyl, geradkettiges oder verzweigtes $C_{1-8}$-Alkyl, $C_{3-6}$-Cycloalkyl, Phenyl oder für Kombinationen davon steht und $R^{14}$ für Wasserstoff, $C_{1-6}$-Alkyl, geradkettiges oder verzweigtes $C_{1-6}$-Alkoxy, Halogen, Nitro oder Cyano steht,

B für folgendes steht:

(a)

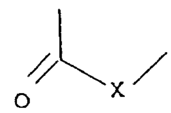

wenn Q vorhanden ist

worin X für $NR^1$ oder O steht und $R^1$ fehlt oder für Wasserstoff, Methyl der Ethyl steht,

(b)

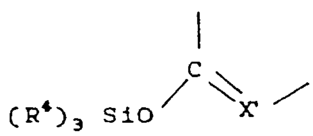

worin X' N bedeutet,

(c)

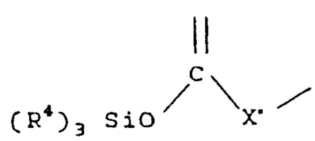

worin X'' O bedeutet,

Z für folgendes steht:

(1) $\beta$-Wasserstoff und $\alpha$-Hydroxy,

(2) $\alpha$-Wasserstoff oder $\alpha$-Hydroxy und

(a)

$$-Alk-\overset{O}{\overset{\|}{C}}-R^8,$$

worin

Alk vorhanden ist oder fehlt und eine geradkettige oder verzweigte Kohlenwasserstoffkette mit 1 bis 12 Kohlenstoffatomen ist und $R^8$ für folgendes steht:

(i) Wasserstoff,

(ii) Hydroxy,

(iii) $C_{1-12}$-Alkyl,

(iv) $NR^9R^{10}$, worin $R^9$ und $R^{10}$ jeweils unabhängig ausgewählt werden aus Wasserstoff, geradkettigem oder verzweigtem $C_{1-12}$-Alkyl, geradkettigem oder verzweigtem $C_{1-12}$-Alkyl, bei dem ein Wasserstoff durch Hydroxy, Carbonsäure oder $C_{1-4}$-Alkylester substituiert ist, $C_{3-6}$-Cycloalkyl, Phenyl, oder $R^9$ und $R^{10}$ zusammengenommen mit dem Stickstoff, an den sie angeknüpft sind,

einen 5- bis 6-gliedrigen gesättigten Ring darstellen, der bis zu einem weiteren Heteroatom umfaßt, ausgewählt aus Sauerstoff und Stickstoff, oder

(v) $OR^{11}$, worin $R^{11}$ für M steht, worin M Wasserstoff oder Alkalimetall bedeutet, oder geradkettiges oder verzweigtes $C_{1-18}$-Alkyl, Benzyl, oder

(b) -(Alk)-$OR^{12}$, worin Alk immer vorhanden ist und die gleiche Bedeutung wie oben besitzt und $R^{12}$ für

(i) Phenyl-$C_{1-6}$-alkylcarbonyl,
(ii) $C_{5-10}$-Cycloalkylcarbonyl,
(iii) Benzoyl oder
(iv) $C_{1-18}$-Alkoxycarbonyl,
(v) Amino oder $C_{1-8}$-Alkyl, substituiertes Amino, Carbonyl, oder
(vi) Wasserstoff steht, mit der Maßgabe, daß Alk eine verzweigte $C_3$-$C_8$-Kette bedeutet,

(3)

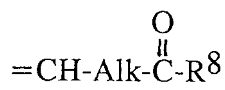

oder =CH-Alk-$OR^{12}$, worin Alk vorhanden ist oder fehlt und dieselbe Bedeutung wie oben besitzt und $R^8$ und $R^{12}$ dieselbe Bedeutung wie oben besitzen und $R^{12}$ ferner für Wasserstoff oder $C_{1-20}$-Alkylcarbonyl steht,

(4)

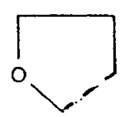

worin die gestrichelte Bindung den 17$\alpha$-Wasserstoff ersetzt,

(5) $\alpha$-Wasserstoff und

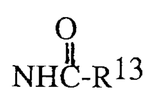,

worin $R^{13}$ für

(a) $C_{1-12}$-Alkyl oder
(b) $NR^9R^{10}$ steht,

(6) $\alpha$-Wasserstoff und Cyano oder

(7) $\alpha$-Wasserstoff und Tetrazolyl,

R' Wasserstoff und Methyl bedeutet,
R'' Wasserstoff oder $\beta$-Methyl bedeutet,
R''' Wasserstoff, $\beta$-Methyl oder Hydroxy bedeutet, dadurch gekennzeichnet, daß ein Lacton oder Lactam, insbesondere ein 3-Keto-4-azasteroid der Formel

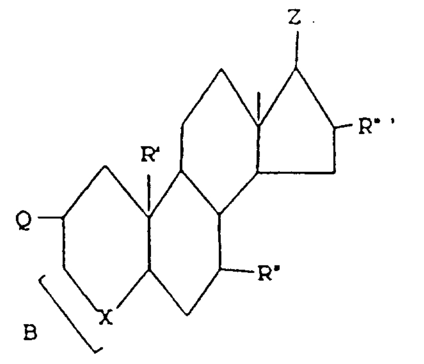

(I)

worin B, Z, X, R', R'', R''' die vorstehend gegebenen Definitionen besitzen, mit einem Silylierungsmittel in Gegenwart eines Chinons umgesetzt wird.

17. Verfahren nach Anspruch 16, bei dem das Silylierungsmittel Bistrimethylsilyltrihalogenacetamid, Bistrimethylsilylacetamid, Hexamethyldisilazan oder Bistrimethylsilylharnstoff ist.

18. Verfahren nach Anspruch 17, bei dem das Chinon

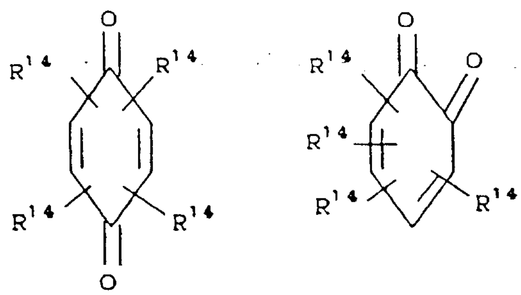

ist, worin $R^{14}$ für Wasserstoff, $C_{1-6}$-Alkyl, $C_{1-6}$-Alkoxy, Halogen, Nitro oder Cyano steht.

19. Verfahren nach Anspruch 18, bei dem das Chinon 2,3-Dichlor-5,6-dicyano-1,4-benzochinon ist.

20. Verfahren nach Anspruch 16, bei dem Q vorhanden ist und $R^4$ für Methyl steht, B für

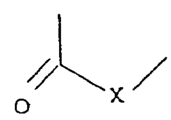

steht, worin X $NR^1$ bedeutet,
R'', R''' für Wasserstoff stehen,
Z für

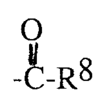

steht, worin

$R^8$ für folgendes steht:

(i) verzweigtes $C_{3-12}$-Alkyl-NH,

(ii) verzweigtes $C_{3-12}$-Alkyl-NH, bei dem ein Wasserstoff mit Hydroxy, Carbonsäure oder $C_{1-4}$-Alkylester substituiert ist,

(iii) verzweigtes -$C_{3-12}$-Alkyl oder

(iv) -$OCH_3$.

**21.** Verfahren nach Anspruch 20, bei dem $R^8$ für -NH-t-Butyl,

$$\overset{CH_2OH}{\overset{|}{-NHC(CH_3)_2}},$$

sec-Butyl, Isobutyl oder -$OCH_3$ steht.

**22.** Verfahren nach Anspruch 21, bei dem $R^1$ für Wasserstoffsteht und $R^8$ -NH-t-Butyl bedeutet.

**23.** Verfahren nach Anspruch 22, bei dem Q für

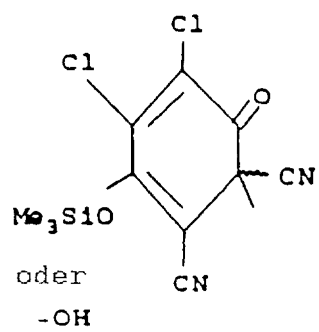

steht.

**24.** Verfahren nach Anspruch 22, bei dem Q für

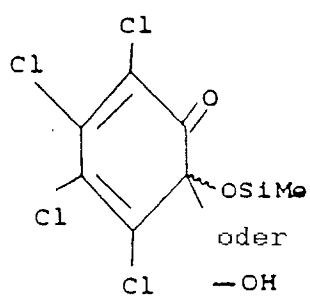

steht.

**Revendications**

**Revendications pour les Etats contractants suivants : AT, BE, CH, LI, DE, FR, GB, IT, LU, NL, SE**

**1.** Procédé pour déshydrogéner un composé de formule

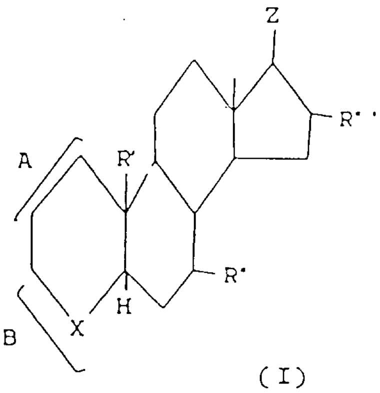

(I)

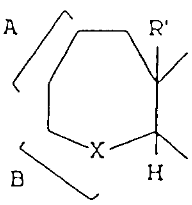

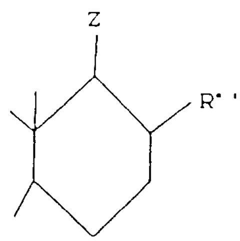

(II) (III)

où la formule (I) peut aussi avoir la structure des formules partielles (II) et/ou (III),
où A est :
(1) $-CH_2-CH_2-$ ;
(2)

$$\begin{array}{l} CH_3 \\ -CH-CH_2- \; ; \\ 1 \;\; 2 \end{array}$$

B est
(1)

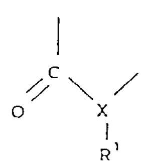

où X est N ou O ; et
$R^1$ est absent ou est
(a) un hydrogène ;

(b) un radical méthyle ou éthyle ;

(c) $NR^2R^3$ où $R^2$ et $R^3$ sont chacun un hydrogène ou un radical méthyle ; ou

(d) un radical cyano ; ou bien

(2)

(a)

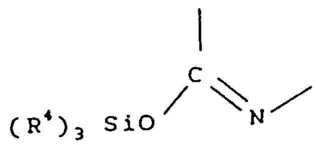

(b)

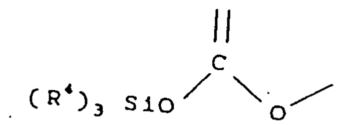

(c)

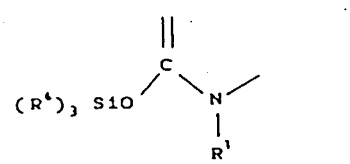

où $R^4$ est l'un quelconque des radicaux méthyle, alkyle en $C_{1-8}$ a chaîne droite ou ramifiée, cycloalkyle en $C_{3-6}$, phényle, ou leurs combinaisons ;

R' est un hydrogène ou le radical méthyle ;

R'' est un hydrogène ou le radical $\beta$-méthyle ;

R''' est un hydrogène ou le radical $\beta$-méthyle ou hydroxyle ;

Z est

(1) un $\beta$-hydrogène ou un radical $\alpha$-hydroxyle ;

(2) un $\alpha$-hydrogène ou un radical $\alpha$-hydroxyle, et

(a)

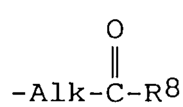

ou Alk est présent ou absent et représente une chaîne hydrocarbonée droite ou ramifiée ayant de 1 à 12 atomes de carbone, et $R^8$ est

(i) un hydrogène,

(ii) un radical hydroxyle,

(iii) un radical alkyle en $C_{1-12}$,

(iv) $NR^9R^{10}$, ou $R^9$ et $R^{10}$, indépendamment l'un de l'autre, sont chacun choisis parmi l'hydrogène ; les radicaux alkyle en $C_{1-12}$ à chaîne droite ou ramifiée ; les radicaux alkyle en $C_{1-12}$ à chaîne droite ou ramifiée dont un hydrogène est substitué par un radical hydroxy, un acide carboxylique ou un ester alkylique en $C_{1-4}$ ; cycloalkyle en $C_{3-6}$ ; phényle ; ou bien encore $R^9$ et $R^{10}$, pris avec l'atome d'azote auquel ils sont liés, représentent un cycle saturé à 5 ou 6 chaînons comprenant jusqu'à un autre hétéroatome choisi parmi l'oxygène et l'azote ; ou bien

(v) $OR^{11}$, où $R^{11}$ est M, où M est un hydrogène ou un métal alcalin, ou encore un radical alkyle en $C_{1-18}$ à chaîne droite ou ramifiée ; benzyle ; ou bien

(b) -(Alk)-$OR^{12}$, où Alk est présent ou absent et a les mêmes significations que ci-dessus ; et $R^{12}$ est

(i) un radical phényle (alkyle en $C_{1-6}$)carbonyle,

(ii) un radical (cycloalkyle en $C_{5-10}$)carbonyle,

(iii) un radical benzyle, ou

(iv) un radical (alcoxy en $C_{1-18}$)carbonyle,

(v) un hydrogène ;

(3)

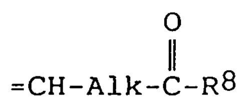

ou bien =CH-Alk-$OR^{12}$, où Alk est présent ou absent et a les mêmes significations que ci-dessus, et $R^8$ et $R^{12}$ ont les mêmes significations que ci-dessus, et $R^{12}$ est aussi un hydrogène ou un radical (alkyle en $C_{1-20}$)carbonyle ;

(4)

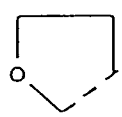

où la ligne en tirets remplace l'hydrogène 17$\alpha$ ;

(5) un $\alpha$-hydrogène, et

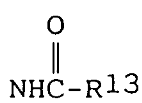

où $R^{13}$ est

(a) un radical alkyle en $C_{1-12}$ ; ou

(b) $NR^9R^{10}$ ;

(6) un $\alpha$-hydrogène et un radical cyano ; ou bien

(7) un $\alpha$-hydrogène et un radical tétrazolyle ; qui consiste à faire réagir le composé avec un agent de silylation en présence d'une quinone pour introduire une double liaison $\Delta^1$.

**2.** Procédé selon la revendication 1, dans lequel l'agent de silylation est un bistriméthylsilyltrihalogénoacétamide, le bistriméthylsilylacétamide, l'hexaméthyldisilazane ou la bistriméthylsilylurée.

**3.** Procédé selon la revendication 2, dans lequel la quinone est

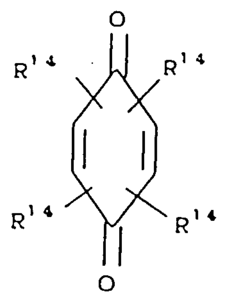

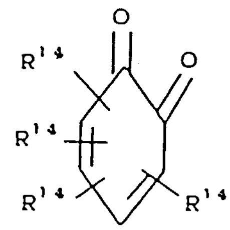

où $R^{14}$ est un hydrogène ou un radical alkyle en $C_{1-6}$, alcoxy en $C_{1-6}$, halogéno, nitro ou cyano.

**4.** Procédé selon la revendication 3, dans lequel la quinone est la 2,3-dichloro-5,6-dicyano-1,4-benzoquinone.

**5.** Procédé selon la revendication 1, dans lequel
A est $-CH_2-CH_2-$
B est

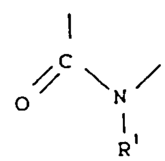

où $R^1$ est :
(a) un hydrogène ;
(b) un radical méthyle ou éthyle ;
R" et R"' sont des hydrogènes ; et
Z est

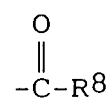

où $R^8$ est
(i) un radical -NH(alkyle ramifie en $C_{3-12}$) ;
(ii) un radical -NH(alkyle ramifie en $C_{3-12}$) ayant un hydrogène substitue par un hydroxy, un acide carboxylique ou un ester alkylique en $C_{1-4}$ ;
(iii) un radical alkyle en $C_{3-12}$ ramifie ; ou encore
(iv) $-OCH_3$

**6.** Procédé selon la revendication 5, dans lequel l'agent de silylation est un bistrimethylsilyltrihalogénoacétamide, le bistriméthylsilylacétamide, l'hexaméthyldisilazane ou la bistriméthylsilylurée.

**7.** Procédé selon la revendication 6, dans lequel l'agent de silylation est le bistriméthylsilyltrifluoroacétamide, et la quinone est la 2,3-dichloro-5,6-dicyano-1,4-benzoquinone.

**8.** Procédé selon la revendication 7, dans lequel $R^1$ est un hydrogène et $R^8$ est le radical -NH-tert-butyle.

**9.** Procédé de déshydrogénation, qui consiste à faire réagir la 17$\beta$-N-(tert-butylcarbamoyl)-4-aza-5$\alpha$-androstane-3-one avec un agent de silylation en présence d'une quinone pour former la 17$\beta$-N-(tert-butylcarbamoyl)-4-aza-5$\alpha$-androst-1-ène-3-one.

**10.** Procédé selon la revendication 9, dans lequel l'agent de silylation est le bistriméthylsilyltrifluoroacétamide, et la quinone est la 2,3-dichloro-5,6-dicyano-1,4-benzoquinone.

**11.** Procédé selon la revendication 1, qui consiste à faire réagir la 23-méthyl-4-aza-21-nor-5$\alpha$-cholane-3,20-dione avec un agent de silylation en présence d'une quinone pour former le compose $\Delta^1$.

**12.** Procédé selon la revendication 1, qui consiste à faire réagir la 22-méthyl-4-aza-21-nor-5$\alpha$-cholane-3,20-dione avec un agent de silylation en présence d'une quinone pour former le composé $\Delta^1$.

**13.** Procédé selon la revendication 1, qui consiste à faire réagir l'acide 3-oxo-4-aza-5$\alpha$-androstane-17$\beta$-carboxylique avec un agent de silylation en présence d'une quinone pour former le composé $\Delta^1$.

**14.** Procédé selon la revendication 1, qui consiste à faire réagir la 17$\beta$-(isopropylcarbonyl)-4-aza-5$\alpha$-androstane-3-one avec un agent de silylation en présence d'une quinone pour former le composé $\Delta^1$.

**15.** Procédé selon la revendication 1, qui consiste à faire réagir la 17$\beta$-(carbométhoxy)-4-aza-5$\alpha$-androstane-3-one avec un agent de silylation en présence d'une quinone pour former le composé $\Delta^1$.

**16.** Composés de formule

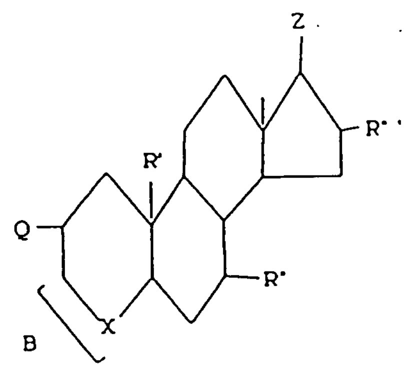

(I)

dans laquelle :

Q est absent ou bien est

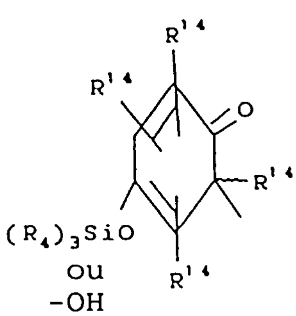

ou
-OH

ou

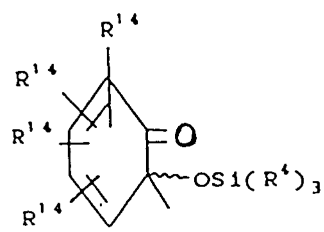

ou
-OH

où $R^4$ est un radical méthyle, alkyle à chaîne droite ou ramifiée en $C_{1-8}$, cycloalkyle en $C_{3-6}$, phényle, ou leurs combinaisons ; et $R^{14}$ est un hydrogène ou un radical alkyle en $C_{1-6}$ à chaîne droite ou ramifiée, alcoxy en $C_{1-6}$, halogéno, nitro ou cyano ;

B est :

(a) quand Q est présent,

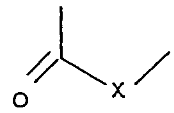

où X est $NR^1$ ou O ; et $R^1$ est absent ou est un hydrogène ou un radical méthyle ou éthyle ;

(b)

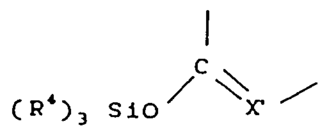

où X' est N ;

(c)

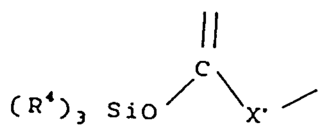

où X'' est O ;

Z est

(1) un $\beta$-hydrogène et un radical $\alpha$-hydroxyle ;

(2) un $\alpha$-hydrogène ou un radical $\alpha$-hydroxyle, et

(a)

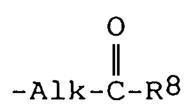

où Alk est présent ou absent et est une chaîne hydrocarbonée à chaîne droite ou ramifiée ayant de 1 à 12 atomes de carbone ; et $R^8$ est

(i) un hydrogène,

(ii) un radical hydroxyle,

(iii) un radical en $C_{1-12}$,

(iv) $NR^9R^{10}$, où $R^9$ et $R^{10}$, indépendamment l'un de l'autre, sont chacun choisis parmi l'hydrogène et les radicaux alkyle à chaîne droite ou ramifiée en $C_{1-12}$ ; alkyle à chaîne droite ou ramifiée en $C_{1-12}$ ayant un hydrogène substitué par un radical hydroxy, acide carboxylique ou ester alkylique en $C_{1-4}$ ; cycloalkyle en $C_{3-6}$ ; ou $R^9$ et $R^{10}$, pris ensemble avec l'azote auquel ils sont liés, représentent un noyau saturé à 5 ou 6 chaînons comprenant jusqu'à un autre hétéroatome choisi parmi l'oxygène et l'azote ; ou bien

(v) $OR^{11}$, où $R^{11}$ est M, où M est un hydrogène ou un métal alcalin, ou encore un radical alkyle en $C_{1-18}$ à chaîne droite ou ramifiée ; benzyle ; ou bien

(b) -(Alk)-$OR^{12}$, où Alk est toujours présent et a les mêmes significations que ci-dessus ; et $R^{12}$ est

(i) un radical phényl-(alkyle en $C_{1-6}$)carbonyle,

(ii) un radical (cycloalkyle en $C_{5-10}$)carbonyle,

(iii) un radical benzoyle, ou bien
(iv) un radical (alcoxy en $C_{1-18}$)carbonyle,
(v) un radical amino ou un radical amino substitué par des radicaux alkyle en $C_{1-8}$, carbonyle, ou
(vi) un hydrogène, du moment que Alk est une chaîne ramifiée en $C_{3-8}$ ;

(3)

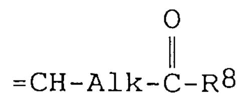

ou encore =CH-Alk-$OR^{12}$, où Alk est présent ou est absent et a les mêmes significations que ci-dessus, et $R^8$ et $R^{12}$ ont les mêmes significations que ci-dessus, et $R^{12}$ est aussi un hydrogène ou un radical (alkyle en $C_{1-20}$)carbonyle ;

(4)

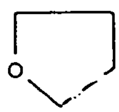

où la liaison en tirets remplace l'hydrogène 17$\alpha$ ;

(5) un $\alpha$-hydrogène et formule

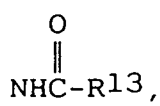

où $R^{13}$ est
(a) un radical alkyle en $C_{1-12}$, ou
(b) $NR^9R^{10}$ ;

(6) un $\alpha$-hydrogène et un radical cyano ; ou encore
(7) un $\alpha$-hydrogène et un radical tétrazolyle ;

R' est un hydrogène ou le radical méthyle ;
R'' est un hydrogène ou le radical $\beta$-méthyle ;
R''' est un hydrogène ou un radical $\beta$-méthyle ou hydroxyle.

**17.** Composés selon la revendication 16, dans lesquels Q est présent et $R^4$ est le radical méthyle ;
B est

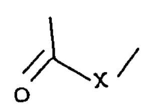

où X est $NR^1$ ;
R'' et R''' sont des hydrogènes ; et
Z est

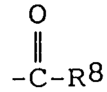

où $R^8$ est
(i) un radical -NH(alkyle ramifié en $C_{3-12}$) ;
(ii) un radical -NH(alkyle ramifié en $C_{3-12}$) ayant un hydrogène substitué par un hydroxy, un acide carboxylique ou un ester alkylique en $C_{1-4}$ ;
(iii) un radical alkyle en $C_{3-12}$ ramifie ; ou encore
(iv) $-OCH_3$

**18.** Composés selon la revendication 17, dans lesquels $R^8$ est l'un des radicaux -NH-tert-butyle,

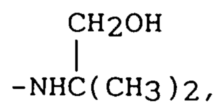

sec-butyle, isobutyle ou $-OCH_3$.

**19.** Composé selon la revendication 18, dans lequel $R^1$ est un hydrogène et $R^8$ est le radical -NH-tert-butyle.

**20.** Composé selon la revendication 9, dans lequel Q est

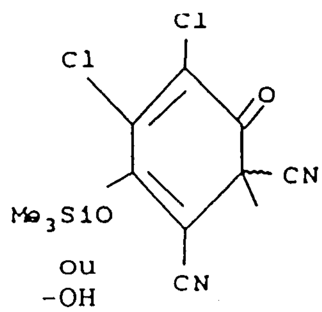

**21.** Composé selon la revendication 19, dans lequel Q est

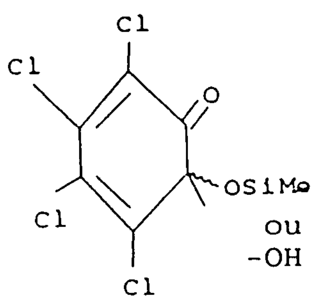

**Revendications pour les Etats contractants suivants : ES, GR**

**1.** Procédé pour déshydrogéner des lactones et des lactames, en particulier des 3-céto-4-azastéroïdes, de formule

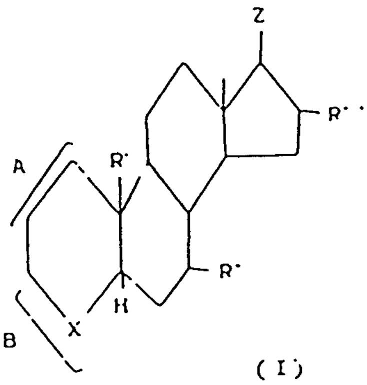

(I)

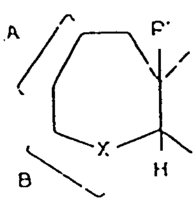

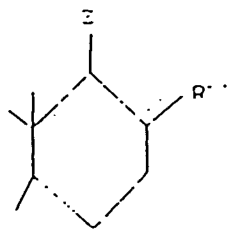

(II) (III)

où la formule (I) peut aussi avoir la structure des formules partielles (II) et/ou (III), où A est :

(1) $-CH_2-CH_2-$ ;

(2)

CH3
-CH-CH2- ;
1 2

B est

(1)

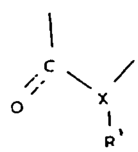

où X est N ou O ; et

$R^1$ est absent ou est

(a) un hydrogène ;

(b) un radical méthyle ou éthyle ;

(c) $NR^2R^3$ où $R^2$ et $R^3$ sont chacun un hydrogène ou un radical méthyle ; ou

(d) un radical cyano ; ou bien

(2)

(a)

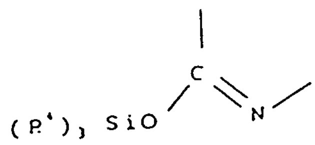

(b)

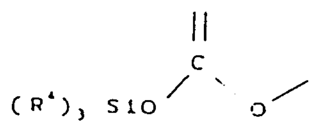

(c)

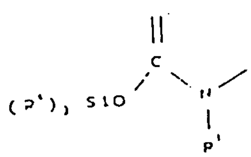

où $R^4$ est l'un quelconque des radicaux méthyle, alkyle en $C_{1-8}$ à chaîne droite ou ramifiée, cycloalkyle en $C_{3-6}$, phényle, ou leurs combinaisons ;

R' est un hydrogène ou le radical méthyle ;

R'' est un hydrogène ou le radical $\beta$-méthyle ;

R''' est un hydrogène ou le radical $\beta$-méthyle ou hydroxyle ;

Z est

(1) un $\beta$-hydrogène ou un radical $\alpha$-hydroxyle ;

(2) un $\alpha$-hydrogène ou un radical $\alpha$-hydroxyle, et

(a)

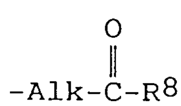

où Alk est présent ou absent et représente une chaîne hydrocarbonée droite ou ramifiée ayant de 1 à 12 atomes de carbone, et $R^8$ est

(i) un hydrogène,

(ii) un radical hydroxyle,

(iii) un radical alkyle en $C_{1-12}$,

(iv) $NR^9R^{10}$, ou $R^9$ et $R^{10}$, indépendamment l'un de l'autre, sont chacun choisis parmi l'hydrogène ; les radicaux alkyle en $C_{1-12}$ à chaîne droite ou ramifiée ; les radicaux alkyle en $C_{1-12}$ à chaîne droite ou ramifiée dont un hydrogène est substitué par un radical hydroxy, un acide carboxylique ou un ester alkylique en $C_{1-4}$ ; cycloalkyle en $C_{3-6}$ ;

phényle ; ou bien encore $R^9$ et $R^{10}$, pris avec l'atome d'azote auquel ils sont liés, représentent un cycle saturé à 5 ou 6 chaînons comprenant jusqu'à un autre hétéroatome choisi parmi l'oxygène et l'azote ; ou bien

(v) $OR^{11}$, où $R^{11}$ est M, où M est un hydrogène ou un métal alcalin, ou encore un radical alkyle en $C_{1-18}$ à chaîne droite ou ramifiée ; benzyle ; ou bien

(b) -(Alk)-$OR^{12}$, où Alk est présent ou absent et a les mêmes significations que ci-dessus ; et $R^{12}$ est

(i) un radical phényle (alkyle en $C_{1-6}$)carbonyle,

(ii) un radical (cycloalkyle en $C_{5-10}$)carbonyle,

(iii) un radical benzyle, ou

(iv) un radical (alcoxy en $C_{1-18}$)carbonyle,

(v) un hydrogène ;

(3)

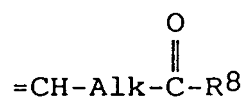

ou bien =CH-Alk-$OR^{12}$, où Alk est présent ou absent et a les mêmes significations que ci-dessus, et $R^8$ et $R^{12}$ ont les mêmes significations que ci-dessus, et $R^{12}$ est aussi un hydrogène ou un radical (alkyle en $C_{1-20}$)carbonyle ;

(4)

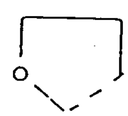

où la ligne en tirets remplace l'hydrogène 17$\alpha$ ;

(5) un $\alpha$-hydrogène, et

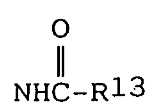

où $R^{13}$ est

(a) un radical alkyle en $C_{1-12}$ ; ou

(b) $NR^9R^{10}$ ;

(6) un $\alpha$-hydrogène et un radical cyano ; ou bien

(7) un $\alpha$-hydrogène et un radical tétrazolyle ; qui consiste à faire réagir le composé avec un agent de silylation en présence d'une quinone pour introduire une double liaison $\Delta^1$.

2. Procédé selon la revendication 1, dans lequel l'agent de silylation est un bistriméthylsilyltrihalogénoacétamide, le bistriméthylsilylacétamide, l'hexaméthyldisilazane ou la bistriméthylsilylurée.

**3.** Procédé selon la revendication 2, dans lequel la quinone est

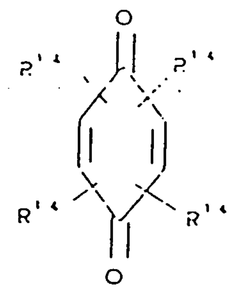

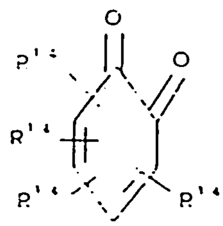

où $R^{14}$ est un hydrogène ou un radical alkyle en $C_{1-6}$, alcoxy en $C_{1-6}$, halogéno, nitro ou cyano.

**4.** Procédé selon la revendication 3, dans lequel la quinone est la 2,3-dichloro-5,6-dicyano-1,4-benzoquinone.

**5.** Procédé selon la revendication 1, dans lequel :
A est $-CH_2-CH_2-$
B est

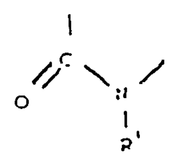

où $R^1$ est :
(a) un hydrogène ;
(b) un radical méthyle ou éthyle ;
R'' et R''' sont des hydrogènes ; et
Z est

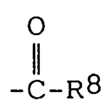

où $R^8$ est
(i) un radical -NH(alkyle ramifié en $C_{3-12}$) ;
(ii) un radical -NH(alkyle ramifié en $C_{3-12}$) ayant un hydrogène substitué par un hydroxy, un acide carboxylique ou un ester alkylique en $C_{1-4}$ ;
(iii) un radical alkyle en $C_{3-12}$ ramifie ; ou encore
(iv) $-OCH_3$

**6.** Procédé selon la revendication 5, dans lequel l'agent de silylation est un bistriméthylsilyltrihalogénoacétamide, le bistriméthylsilylacétamide, l'hexaméthyldisilazane ou la bistriméthylsilylurée.

**7.** Procédé selon la revendication 6, dans lequel l'agent de silylation est le bistriméthylsilyltrifluoroacétamide, et la quinone est la 2,3-dichloro-5,6-dicyano-1,4-benzoquinone.

**8.** Procédé selon la revendication 7, dans lequel $R^1$ est un hydrogène et $R^8$ est le radical -NH-tert-butyle.

**9.** Procédé de déshydrogénation, qui consiste à faire réagir la 17$\beta$-N-(tert-butylcarbamoyl)-4-aza-5$\alpha$-androstane-3-one avec un agent de silylation en présence d'une quinone pour former la 17$\beta$-N-(tert-butylcarbamoyl)-4-aza-5$\alpha$-androst-1-ène-3-one.

**10.** Procédé selon la revendication 9, dans lequel l'agent de silylation est le bistriméthylsilyltrifluoroacétamide, et la quinone est la 2,3-dichloro-5,6-dicyano-1,4-benzoquinone.

**11.** Procédé selon la revendication 1, qui consiste à faire réagir la 23-méthyl-4-aza-21-nor-5$\alpha$-cholane-3,20-dione avec un agent de silylation en présence d'une quinone pour former le composé $\Delta^1$.

**12.** Procédé selon la revendication 1, qui consiste à faire réagir la 22-méthyl-4-aza-21-nor-5$\alpha$-cholane-3,20-dione avec un agent de silylation en présence d'une quinone pour former le composé $\Delta^1$.

**13.** Procédé selon la revendication 1, qui consiste à faire réagir l'acide 3-oxo-4-aza-5$\alpha$-androstane-17$\beta$-carboxylique avec un agent de silylation en présence d'une quinone pour former le composé $\Delta^1$.

**14.** Procédé selon la revendication 1, qui consiste à faire réagir la 17$\beta$-(isopropylcarbonyl)-4-aza-5$\alpha$-androstane-3-one avec un agent de silylation en présence d'une quinone pour former le composé $\Delta^1$.

**15.** Procédé selon la revendication 1, qui consiste à faire réagir la 17$\beta$-(carbométhoxy)-4-aza-5$\alpha$-androstane-3-one avec un agent de silylation en présence d'une quinone pour former le composé $\Delta^1$.

**16.** Procédé pour préparer des composés ayant la formule suivante :

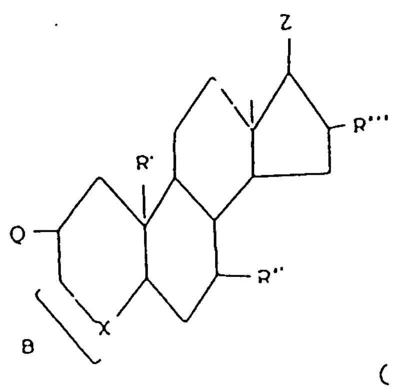

(I)

dans laquelle

Q est absent ou bien est

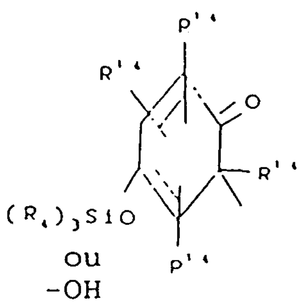

ou

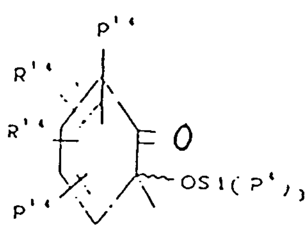

ou -OH ou -OH

où $R^4$ est un radical méthyle, alkyle à chaîne droite ou ramifiée en $C_{1-8}$, cycloalkyle en $C_{3-6}$, phényle, ou leurs combinaisons ; et $R^{14}$ est un hydrogène ou un radical alkyle en $C_{1-6}$ à chaîne droite ou ramifiée, alcoxy en $C_{1-6}$, halogéno, nitro ou cyano ;

B est :

(a) quand Q est présent,

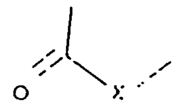

où X est $NR^1$ ou O ; et $R^1$ est absent ou est un hydrogène ou un radical méthyle ou éthyle ;

(b)

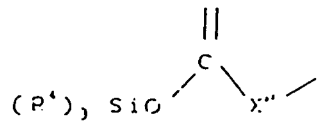

où X' est N ;

(c)

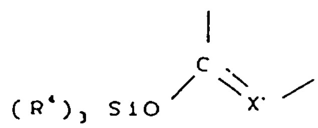

où X'' est O ;

Z est

(1) un $\beta$-hydrogène et un radical $\alpha$-hydroxyle ;

(2) un $\alpha$-hydrogène ou un radical $\alpha$-hydroxyle,

et

(a)

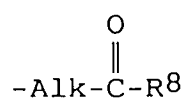

où Alk est présent ou absent et est une chaîne hydrocarbonée à chaîne droite ou ramifiée ayant de 1 à 12 atomes de carbone ; et $R^8$ est

(i) un hydrogène,

(ii) un radical hydroxyle,

(iii) un radical en $C_{1-12}$,

(iv) $NR^9R^{10}$, où $R^9$ et $R^{10}$, indépendamment l'un de l'autre, sont chacun choisis parmi l'hydrogène et les radicaux alkyle à chaîne droite ou ramifiée en $C_{1-12}$ ; alkyle à chaîne droite ou ramifiée en $C_{1-12}$ ayant un hydrogène substitué par un radical hydroxy, acide carboxylique ou ester alkylique en $C_{1-4}$ ; cycloalkyle en $C_{3-6}$ ; ou $R^9$ et $R^{10}$, pris ensemble avec l'azote auquel ils sont liés, représentent un noyau saturé à 5 ou 6 chaînons comprenant jusqu'à un autre hétéroatome choisi parmi l'oxygène et l'azote ; ou bien

(v) $OR^{11}$, où $R^{11}$ est M, où M est un hydrogène ou un métal alcalin, ou encore un radical alkyle en $C_{1-18}$ à chaîne droite ou ramifiée ; benzyle ; ou bien

(b) -(Alk)-$OR^{12}$, où Alk est toujours présent et a les mêmes significations que ci-dessus ; et $R^{12}$ est

(i) un radical phényl-(alkyle en $C_{1-6}$)carbonyle,

(ii) un radical (cycloalkyle en $C_{5-10}$)carbonyle,

(iii) un radical benzoyle, ou bien

(iv) un radical (alcoxy en $C_{1-18}$)carbonyle,
(v) un radical amino ou un radical aminosubstitué par des radicaux alkyle en $C_{1-8}$, carbonyle, ou
(vi) un hydrogène, du moment que Alk est une chaîne ramifiée en $C_{3-8}$ ;

(3)

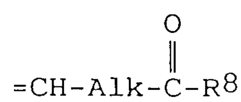

ou encore $=CH\text{-}Alk\text{-}OR^{12}$, où Alk est présent ou est absent et a les mêmes significations que ci-dessus, et $R^8$ et $R^{12}$ ont les mêmes significations que ci-dessus, et $R^{12}$ est aussi un hydrogène ou un radical (alkyle en $C_{1-20}$)carbonyle ;

(4)

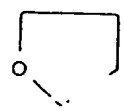

où la liaison en tirets remplace l'hydrogène 17$\alpha$ ;

(5) un $\alpha$-hydrogène et formule

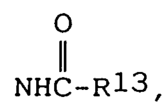

où $R^{13}$ est
(a) un radical alkyle en $C_{1-12}$, ou
(b) $NR^9R^{10}$ ;

(6) un $\alpha$-hydrogène et un radical cyano ; ou encore
(7) un $\alpha$-hydrogène et un radical tétrazolyle ;
R' est un hydrogène ou le radical méthyle ;
R'' est un hydrogène ou le radical $\beta$-méthyle ;
R''' est un hydrogène ou un radical $\beta$-méthyle ou

hydroxyle, caractérisé en ce qu'il consiste à faire réagir une lactone ou un lactame, en particulier un 3-céto-4-azastéroïde de formule

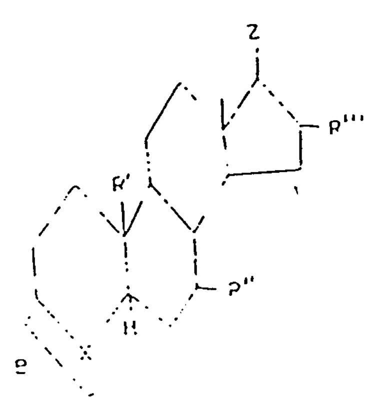

où B, Z, X, R' ; R'' et R''' sont tels que définis ci-dessus, avec un agent de silylation et en présence d'une quinone.

**17.** Procédé selon la revendication 16, dans lequel l'agent de silylation est un bistriméthylsilyltrihalogénoacétamide, le bistriméthylsilylacétamide, l'hexaméthyldisilazane ou la bistriméthylsilylurée.

**18.** Procédé selon la revendication 17, dans lequel la quinone est :

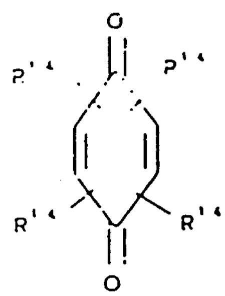

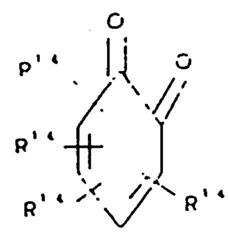

où $R^{14}$ est un hydrogène ou un radical en $C_{1-6}$, alcoxy en $C_{1-6}$, halogéno, nitro ou cyano.

**19.** Procédé selon la revendication 18, dans lequel la quinone est la 2,3-dichloro-5,6-dicyano-1,4-benzoquinone.

**20.** Procédé selon la revendication 16, dans lequel Q est présent et $R^4$ est un radical méthyle ;
B est

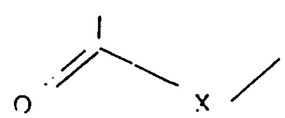

où X est $NR^1$ ;
R'' et R''' sont des hydrogènes ; et
Z est

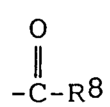

où $R^8$ est
(i) un radical -NH(alkyle ramifié en $C_{3-12}$) ;
(ii) un radical -NH(alkyle ramifié en $C_{3-12}$) ayant un hydrogène substitué par un hydroxy, un acide carboxylique ou un ester alkylique en $C_{1-4}$ ;
(iii) un radical alkyle en $C_{3-12}$ ramifié ; ou encore
(iv) $-OCH_3$

**21.** Procédé selon la revendication 20, dans lequel $R^8$ est l'un des radicaux -NH-tert-butyle,

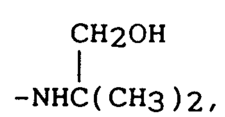

sec-butyle, isobutyle ou $-OCH_3$.

**22.** Procédé selon la revendication 21, dans lequel $R^1$ est un hydrogène et $R^8$ est le radical -NH-tert-butyle.

23. Procédé selon la revendication 22, dans lequel Q est

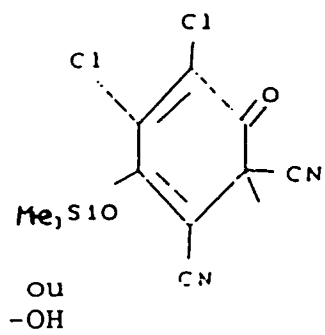

24. Procédé selon la revendication 22, dans lequel Q est